# EUROPEAN PATENT APPLICATION

(11) **EP 3 275 384 A1**
(43) Date of publication of application: **31.01.2018**
(21) Application number: 16768528.8
(22) Date of filing: 14.03.2016
(51) Int. Cl.: A61B 17/34

(54) **ENDOSCOPIC SURGICAL DEVICE**

(30) Priority: 23.03.2015 US 201562136891 P
(71) Applicant: Fujifilm Corporation, Minato-ku Tokyo 106-8620 (JP)
(72) Inventor: KUWAE, Toshiharu, Ashigarakami-gun Kanagawa 258-8538 (JP); YAMAKAWA, Shinichi, Ashigarakami-gun Kanagawa 258-8538 (JP)
(74) Representative: Klunker IP Patentanwälte PartG mbB
(86) International application number: PCT/JP2016/058009
(87) International publication number: WO 2016/152625

(57) **Abstract**

An endoscopic surgical device that can suitably puncture a body wall with an overtube that having two insertion passages is provided. In a case where a body wall is punctured with the overtube 300 that has two insertion passages through which medical instruments are inserted and that has openings 312 and 316 of the insertion passages in a distal end surface 304, distal end parts 606 and 614 of two needle parts of an inner needle are disposed to protrude from the openings 312 and 316 in a case where the inner needle is mounted. Accordingly, a distal end portion of the overtube 300 has a tapered shape, cutting blade parts 632, 634, and 650 are disposed in the same straight line as seen from a distal end side, and an insertion load is reduced.

## Description

### BACKGROUND OF THE INVENTION

### 1. Field of the Invention

The present invention relates to an endoscopic surgical device, and particularly, relates to an endoscopic surgical device that punctures a body wall in a state where an overtube having insertion passages and an inner needle inserted through the overtube are combined together.

### 2. Description of the Related Art

In recent years, since invasion to a patient is small compared to surgery in which a laparotomy, a thoracotomy, or the like is performed, endoscopic surgery using endoscopes (hard endoscopes), such as a laparoscope, is widely performed. In the endoscopic surgery, a plurality of holes are made in a patient's body wall, an endoscope is inserted into a body cavity from one hole of them, and a treatment tool is inserted into the body cavity from another hole. Then, treatment of living body tissue is performed with the treatment tool while observing the living body tissue within the body cavity with the endoscope.

Additionally, an overtube having insertion passages through which medical instruments, such as an endoscope and a treatment tool, are inserted is installed in a hole of a body wall through which the medical instruments are inserted into a body cavity. The formation of the hole of the body wall and the installation of the overtube into the hole are performed by the puncturing of the overtube into the body wall. In that case, an inner needle is mounted on each overtube as shown in JP2009-273891A and JP1993-161660A (JP-H05-161660A) and the like. The inner needle has a needle part through which the overtube is inserted, and a tapered distal end part of the needle part is disposed to protrude from a distal end of the overtube. JP2009-273891A also discloses that a cutting blade (blade) is formed at the distal end part of the needle part.

Then, after a body wall is punctured with the overtube on which the inner needle is mounted, the inner needle is extracted from the overtube, a hole is formed in the body wall, and the overtube is installed in the hole.

Generally, in the endoscopic surgery, one or a plurality of treatment tools are used simultaneously with the endoscope. Therefore, since it is difficult for one operator to simultaneously operate the endoscope and the plurality of treatment tools, for example, a task of operating treatment tools using both hands by the operator while making an assistant called an endoscopic technician operate the endoscope is normally performed.

In this way, in the endoscopic surgery, it is general that the operator's hands are bound by the operation of the treatment tool, and the operation of the endoscope is performed by the assistant. Therefore, in a case where the observation position of the endoscope is changed, the operator should serially give instructions to the assistant. Hence, the task of correctly directing the orientation of the endoscope to a direction desired by the operator is difficult, and stress is likely to be imposed on the operator. Additionally, since the assistant performs an operation after the operator issues an instruction, there is a tendency that surgery time is likely to be prolonged. Additionally, the assistant should operate the endoscope so as not to interfere with an operator's procedure, and the operation is likely to become complicated.

In contrast, the applicant of the present application suggests a technique in which an endoscope and a treatment tool are combined together by an overtube, and if the treatment tool is moved forward and backward, the endoscope is also moved forward and backward in an interlocking manner with this movement of the treatment tool (refer to WO2013/176167A). Specifically, the overtube that guides an insertion part of the endoscope and an insertion part of the treatment tool into a body cavity includes a tubular overtube body that has an endoscope insertion passage and a treatment tool insertion passage through which the insertion part of the endoscope and the insertion part of the treatment tool are inserted, respectively.

A coupling mechanism, which has coupling parts that are respectively coupled to the insertion part of the endoscope and the insertion part of the treatment tool and which is movable forward and backward in an axial direction, is provided inside the overtube body. If the insertion part of the treatment tool is moved forward and backward in the axial direction by the coupling mechanism, the insertion part of the endoscope also moves forward and backward in the axial direction in an interlocking manner with this.

By virtue of such an overtube, the number of holes made in the patient's body wall can be reduced, the invasion to the patient can be suppressed, and the visual field of the endoscope can be easily changed while an operator operates the treatment tool without asking for an assistant's help.

### SUMMARY OF THE INVENTION

Meanwhile, the inner needle described in JP2009-273891A and JP1993-161660A (JP-H05-161660A) is used for an overtube of a single hole having one insertion passage, and JP2009-273891 and JP1993-161660A (JP-H05-161660A) do not suggest an inner needle that is suitable for an overtube having two insertion passages as suggested in WO2013/176167A by the applicant of the present application.

Additionally, as the distal end shapes (distal end structures) of the overtube and the inner needle in a state where the overtube and the inner needle are combined together, it is desirable that the insertion power (penetrating power) required for the puncturing (penetration) of the overtube into the body wall is small and it is easy to perform puncturing. A distal end shape in a state where the overtube and the inner needle are combined together is determined by the distal end shape of one needle part of the inner needle through which the overtube is inserted, in the case of the overtube of the single hole. However, in the case of the overtube having the two insertion passages as suggested in WO2013/176167A by the applicant of the present application, the distal end shape is based on the distal end shapes of two needle parts of the inner needle through which the overtube is inserted and the shape of a distal end surface of the overtube.

Hence, in the case of the overtube having the two insertion passages, even if a distal end shape (distal end shapes) of one or both of the two needle parts of the inner needle is (are) formed in the same shape as that of the inner needle of the overtube of the single hole, this is not always suitable as a combined distal end shape of the overtube and the inner needle.

The invention has been made in view of such circumstances, and an object thereof is to provide an endoscopic surgical device that can suitably puncture a body wall with an overtube having two insertion passages.

In order to achieve the above object, an endoscopic surgical device according to an aspect of the invention is an endoscopic surgical device that has an overtube and an inner needle inserted through the overtube and punctures a body wall in a state where the overtube and the inner needle are combined together. The overtube includes an overtube body having a distal end, a proximal end, and a longitudinal axis, a first distal end opening and a second distal end opening provided at a distal end of the overtube body, a first proximal end opening and a second proximal end opening provided at a proximal end of the overtube body, a first insertion passage that is provided along the longitudinal axis of the overtube body and allows the first distal end opening and the first proximal end opening to communicate with each other, a second insertion passage that is provided along the longitudinal axis of the overtube body and allows the second distal end opening and the second proximal end opening to communicate with each other. The inner needle includes a first needle part that has a first distal end part and is inserted through the first insertion passage, a second needle part that has a second distal end part and is inserted through the second insertion passage, a first cutting blade part that is formed at the first distal end part, and has a length component orthogonal to the longitudinal axis, a second cutting blade part that is formed at the second distal end part and has a length component orthogonal to the longitudinal axis, a positioning part that defines a position of the first distal end part with respect to the first distal end opening and a position of the second distal end part with respect to the second distal end opening in a state where the overtube and the inner needle are combined together. The first cutting blade part and the second cutting blade part are disposed along the same straight line as each other when the first cutting blade part and the second cutting blade part are projected on a plane perpendicular to the longitudinal axis.

According to this aspect, the cutting blade parts that are respectively formed in the distal end parts of the two needle parts of the inner needle in a state where the overtube and the inner needle are combined together form cutting blade parts along the same straight line. By virtue of the cutting blade parts, it is possible to reduce an insertion load without greatly impairing a tearing action onto a body wall. Additionally, the insertion power in a case where a body wall is punctured with the overtube can be made small, and the puncturing of the overtube can be made easy. In addition, the expression "along the same straight line" includes not only a case where the first cutting blade part and the second cutting blade part are on the same straight line, but also a case where the first cutting blade part and the second cutting blade part are not on the same straight line but are parallel to each other and a case where the first cutting blade part and the second cutting blade part are not on the same straight line and are not parallel to each other but are substantially parallel to each other.

In the endoscopic surgical device according to another aspect of the invention, it is possible to adopt an aspect in which the first cutting blade part and the second cutting blade part are disposed on the same straight line as each other when the first cutting blade part and the second cutting blade part are projected on the plane perpendicular to the longitudinal axis.

In the endoscopic surgical device according to still another aspect of the invention, it is possible to adopt an aspect in which the overtube body has a tapered part that is tapered toward a distal end, and the tapered part has the second distal end opening, and the first distal end opening disposed closer to a proximal end side than the second distal end opening.

According to this aspect, the entire shape of the distal end portion of the overtube in a state where the overtube and the inner needle are combined together becomes a tapered shape, and consequently, it is easy to perform the puncturing.

In the endoscopic surgical device according to still another aspect of the invention, it is possible to adopt an aspect in which the second distal end opening is open in a direction perpendicular to the longitudinal axis, and the first distal end opening is open in a direction oblique to the longitudinal axis.

In the endoscopic surgical device according to a still further aspect of the invention, it is possible to adopt an aspect in which the second distal end part has an inclined surface that is tapered toward the distal end, and the inclined surface is provided at a position where the inclined surface protrudes from the second distal end opening when being positioned by the positioning part, and a pair of the second cutting blade parts is provided on the inclined surface, and the pair of second cutting blade parts are disposed at positions that become symmetrical to each other with respect to a central axis of the second needle part.

In the endoscopic surgical device according to a still further aspect of the invention, it is possible to adopt an aspect in which the first distal end part has a distal end surface disposed along an opening surface of the first distal end opening when being positioned by the positioning part, and the first cutting blade part is provided on the distal end surface.

In addition, the expression "along the opening surface" includes not only a case where the distal end surface of the first distal end part and the opening surface of the first distal end opening become parallel to each other and flush (there is no level difference between the distal end surface of the first distal end part and the opening surface of the first distal end opening) with each other, but also a case where the distal end surface of the first distal end part is disposed at a position closer to the proximal end side or the distal end side than the opening surface of the first distal end opening or a case where the distal end surface and the opening surface are substantially parallel to each other. In this way, if the distal end surface of the first distal end part is disposed along the opening surface of the first distal end opening, the above-described effect of reducing the insertion load can be made more remarkable.

In the endoscopic surgical device according to a still further aspect of the invention, it is possible to adopt an aspect in which the overtube includes the interlocking member that is movable forward and backward inside the overtube body, and the interlocking member has a first coupling part coupled to a first insertion part of a first medical instrument inserted through the first insertion passage, and a second coupling part coupled to a second insertion part of a second medical instrument inserted through the second insertion passage.

In the endoscopic surgical device according to a still further aspect of the invention, it is possible to adopt an aspect in which the interlocking member has a non-sensing region where the forward and backward movement of any one of the first insertion part and the second insertion part does not interlock with the forward and backward movement of the other of the first insertion part and the second insertion part, and a sensing region where the forward and backward movement of any one of the first insertion part and the second insertion part interlocks with the forward and backward movement of the other of the first insertion part and the second insertion part.

In the endoscopic surgical device according to a still further aspect of the invention, it is possible to adopt an aspect in which the first insertion passage is an endoscope insertion passage through which an endoscope is inserted, and the second insertion passage is a treatment tool insertion passage through which a treatment tool is inserted.

In the endoscopic surgical device according to a still further aspect of the invention, it is possible to adopt an aspect in which an internal diameter of the second insertion passage is larger than an internal diameter of the first insertion passage.

According to the invention, the endoscopic surgical device that can suitably puncture a body wall with the overtube having the two insertion passages can be provided.

### BRIEF DESCRIPTION OF THE DRAWINGS

Fig. 1 is a schematic block diagram of an endoscopic surgical device according to the invention.
Fig. 2 is a plan view illustrating a distal end surface of the endoscope insertion part.
Fig. 3 is a perspective view illustrating a state where a sheathing tube is fitted to the overtube.
Fig. 4 is an external perspective view illustrating the overtube.
Fig. 5 is a horizontal cross-sectional view illustrating the internal structure of the overtube.
Fig. 6 is an enlarged view of a portion where the slider is disposed in Fig. 5.
Fig. 7 is a perspective view illustrating the overtube with a long tubular body in a long tubular overtube part omitted.
Fig. 8 is a perspective view illustrating the overtube with the long tubular body in the long tubular overtube part omitted.
Fig. 9 is a perspective view illustrating only a partition wall member.
Fig. 10 is a perspective view illustrating only a coupling ring.
Fig. 11 is a cross-sectional view when viewed from arrow A-A in Fig. 6.
Fig. 12 is a perspective view illustrating a portion of the overtube in the state of Fig. 7.
Fig. 13 is a perspective view illustrating the overtube of Fig. 12 with the coupling ring omitted.
Fig. 14 is a perspective view illustrating the overtube of Fig. 13 from a right side.
Fig. 15 is an explanatory view illustrating a state of the operation in a case where the diseased site within the patient's body cavity is treated using the endoscopic surgical device.
Fig. 16 is an explanatory view illustrating a state of the operation in a case where the diseased site within the patient's body cavity is treated using the endoscopic surgical device.
Fig. 17 is a cross-sectional view illustrating one state of a slider.
Fig. 18 is a cross-sectional view illustrating one state of the slider.
Fig. 19 is a cross-sectional view illustrating one state of the slider.
Fig. 20 is an external perspective view in another embodiment of the overtube.
Fig. 21 is a perspective view illustrating the overtube of Fig. 20 with the long tubular body of the long tubular overtube part omitted.
Fig. 22 is a perspective view illustrating the overtube of Fig. 20 with the long tubular body of the long tubular overtube part omitted.
Fig. 23 is a perspective view illustrating a portion of the overtube of Fig. 20.
Fig. 24 is a perspective view illustrating an inner needle to be mounted on the overtube.
Fig. 25 is a side view illustrating the overtube on which the inner needle is mounted.
Fig. 26 is a perspective view illustrating a state in a case where the inner needle is mounted on the overtube.
Fig. 27 is a perspective view illustrating a distal end portion of the overtube.
Fig. 28 is a plan view illustrating the distal end portion of the overtube from an upper side.
Fig. 29 is a front view illustrating the distal end portion of the overtube from a distal end side.
Fig. 30 is a perspective view illustrating the distal end portion of the overtube on which the inner needle is mounted.
Fig. 31 is a plan view illustrating the distal end portion of the overtube, on which the inner needle is mounted, from the upper side.
Fig. 32 is a front view illustrating the distal end portion of the overtube, on which the inner needle is mounted, from the distal end side.
Fig. 33 is a plan view illustrating the periphery of a distal end part of a long needle part of the inner needle from the upper side.
Fig. 34 is a front view illustrating the periphery of the distal end part of the long needle part of the inner needle from the distal end side.
Fig. 35 is a plan view illustrating the periphery of a distal end part of a short needle part of the inner needle from the upper side.
Fig. 36 is a front view illustrating the periphery of the distal end part of the short needle part of the inner needle from the distal end side.
Fig. 37A is a view illustrating the shape of the distal end portion of the overtube in a state where the inner needle is mounted in the present embodiment.
Fig. 37B is a view illustrating the shape of a distal end portion of the overtube in a state where the inner needle is mounted in a first comparison embodiment.
Fig. 37C is a view illustrating the shape of a distal end portion of the overtube in a state where the inner needle is mounted in a second comparison embodiment.
Fig. 38 is a view in which the magnitudes of penetrating power according to three embodiments of Figs. 37A to 37C are compared with each other.
Fig. 39A is a plan view illustrating the shape of the distal end part of the overtube where the inner needle is mounted from the upper side in a simplified manner and is a view illustrating a form of a cutting blade part of the short needle part.
Fig. 39B is a plan view illustrating the shape of the distal end part of the overtube where the inner needle is mounted from the upper side in a simplified manner and is a view illustrating a different form of a cutting blade part of the short needle part.
Fig. 39C is a plan view illustrating the shape of the distal end part of the overtube where the inner needle is mounted from the upper side in a simplified manner and is a view illustrating a different form of a cutting blade part of the short needle part.
Fig. 39D is a plan view illustrating the shape of the distal end part of the overtube where the inner needle is mounted from the upper side in a simplified manner and is a view illustrating a different form of a cutting blade part of the short needle part.
Fig. 39E is a plan view illustrating the shape of the distal end part of the overtube where the inner needle is mounted from the upper side in a simplified manner and is a view illustrating a different form of the cutting blade part of the short needle part.
Fig. 40 is a perspective view illustrating an inner needle corresponding to the overtube of Fig. 20.
Fig. 41 is a front view illustrating the distal end portion of the overtube of Fig. 20, on which the inner needle of Fig. 40 is mounted, from the distal end side.

### DESCRIPTION OF THE PREFERRED EMBODIMENTS

Preferred embodiments of the invention will be described below in detail according to the accompanying drawings. In addition, any of the drawings may illustrate main parts in an exaggerated manner for description, and may have dimensions different from actual dimensions.

Preferred embodiments of the invention will be described below in detail according to the accompanying drawings. In addition, any of the drawings may illustrate main parts in an exaggerated manner for description, and may have dimensions different from actual dimensions.

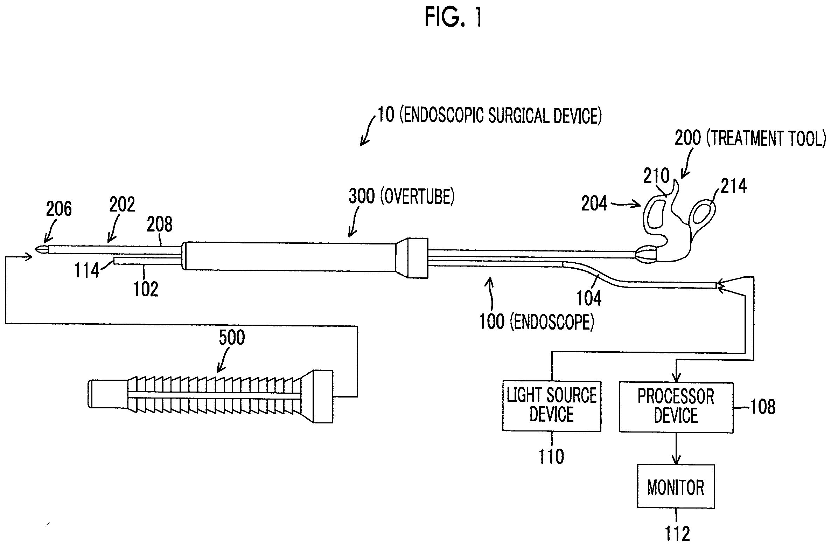

Fig. 1 is a schematic block diagram of an endoscopic surgical device according to the invention. As illustrated in Fig. 1, an endoscopic surgical device 10 includes an endoscope 100 that is one form of a first medical instrument having a first insertion part inserted into a body cavity and observes the inside of a patient's body cavity, a treatment tool 200 that is one form of a second medical instrument having a second insertion part inserted into the body cavity and examines or treats a diseased site within the patient's body cavity, an overtube 300 that is inserted into the body cavity through a body wall and guides an insertion part 102 of the endoscope 100, which is a first insertion part, and an insertion part 202 of a treatment tool 200, which is a second insertion part, to the inside of the body cavity, and a sheathing tube 500 fitted to the overtube 300.

The endoscope 100 is, for example, a hard endoscope, such as a laparoscope, and includes an insertion part 102 (hereinafter referred to as "endoscope insertion part 102") that is inserted into a body cavity, and that has an outer peripheral part surrounded by an elongated hard tubular body, and a cable part 104 that is consecutively installed on a proximal end side of the endoscope insertion part 102 and that has an outer peripheral part surrounded by an elongated flexible tubular body.

The cable part 104 indicates a flexible cable portion in which a wire rod, such as a cable or a light guide, which extends from a proximal end of the endoscope insertion part 102, is housed by covering the wire rod with, for example, a flexible insulating member, such as polyvinyl chloride.

A connector (not illustrated) is provided at an end of the cable part 104 on its extension destination, and each of a processor device 108 that is a control device and a light source device 110 is detachably connected to the cable part via the connector. Additionally, the processor device 108 is connected to a monitor 112 via a cable.

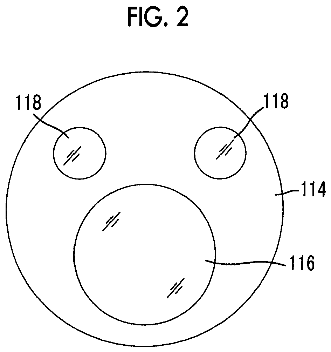

As illustrated in Fig. 2, a distal end surface 114 of the endoscope insertion part 102 is provided with an observation window 116 and illumination windows 118 and 118.

A distal end of the endoscope insertion part 102 is provided with an observation part, and an observation window 116 is provided as a constituent element of the observation part. Additionally, as constituent elements of the observation part, and an objective lens of an observation optical system, and a solid image pickup element, such as a charge coupled device (CCD) image sensor or a complementary metal oxide semiconductor (CMOS) image sensor, which is disposed at an image pickup position of the objective lens, are disposed behind the observation window 116.

A signal cable (not illustrated) connected to this solid image pickup element is inserted through the endoscope insertion part 102 and the cable part 104 of Fig. 1, is provided to extend up to the connector (not illustrated), and is connected to the processor device 108. An observation image picked up from the observation window 116 is formed on a light-receiving surface of the image pickup element, and is converted into electrical signals (image pickup signals), and the electrical signals are output to the processor device 108 via the signal cable and are converted into video signals. Then, the video signals are output to the monitor 112 connected to the processor device 108, and the observation image (endoscopic image) is displayed on a screen of the monitor 112.

An emission end of the light guide (not illustrated) is disposed behind the illumination windows 118 and 118 of Fig. 2 to constitute an illumination part. The light guide is inserted through the endoscope insertion part 102 and the cable part 104 of Fig. 1 and has an incident end disposed within the connector (not illustrated). Hence, by coupling the connector to the light source device 110, the illumination light radiated from the light source device 110 is transmitted to the illumination windows 118 and 118 via the light guide, and is radiated forward from the illumination windows 118 and 118. In addition, in Fig. 2, the two illumination windows 118 and 118 are disposed on the distal end surface 114 of the endoscope insertion part 102. However, the number of illumination windows 118 is not limited, and the number thereof may be one or may be three or more. Additionally, the endoscope 100 may not include the illumination part.

As illustrated in Fig. 1, the treatment tool 200 consists of, for example, forceps, and includes an elongated insertion part 202 (hereinafter referred to as a "treatment tool insertion part 202") that is inserted into a body cavity, an operating part 204 that is provided on the proximal end side of the treatment tool insertion part 202 and is gripped by an operator, and a treatment part 206 that is provided at a distal end of the treatment tool insertion part 202 and is operable by the operation of the operating part 204.

The treatment tool insertion part 202 is provided with a tubular sheath 208, and an operating shaft (not illustrated) that is inserted into the sheath 208 so as to be movable in the direction of an axial center. Moreover, the operating part 204 is provided with a fixed handle 210, and a movable handle 214 that is coupled to the fixed handle 210 in a rotationally movable manner via a rotational movement pin. A proximal end part of the operating shaft is coupled to the movable handle 214.

The treatment part 206 is provided with a pair of gripping members that is openable and closable. The gripping members are coupled to a distal end part of the operating shaft via a driving mechanism (not illustrated). With the rotational movement operation of the movable handle 214 of the operating part 204, the gripping members of the treatment part 206 are opened and closed via the operating shaft and the driving mechanism.

In addition, the treatment tool 200 is not limited to the forceps, and may be, for example, other treatment tools, such as a laser probe, a suture device, an electric scalpel, a needle holder, an ultrasonic device, and an aspirator.

As illustrated in Fig. 1, the overtube 300 allows the endoscope insertion part 102 and the treatment tool insertion part 202, which are inserted thereinto from the proximal end side, to be inserted therethrough and delivered from the distal end side. By inserting the overtube 300 into a body wall and having a proximal end side thereof disposed outside of the body and a distal end side thereof disposed within the body cavity, the endoscope insertion part 102 and the treatment tool insertion part 202 are guided into the body cavity with one overtube 300. Additionally, the overtube 300 includes an interlocking function of moving the endoscope insertion part 102 and the treatment tool insertion part 202 forward and backward in an interlocking manner as will be described below in detail. For example, the endoscope insertion part 102 can also be moved forward and backward by the forward and backward movement operation of only the treatment tool insertion part 202, and a suitable endoscopic image can be obtained without performing the forward and backward movement operation of the endoscope insertion part 102. The details of the configuration and working of the overtube 300 will be described below.

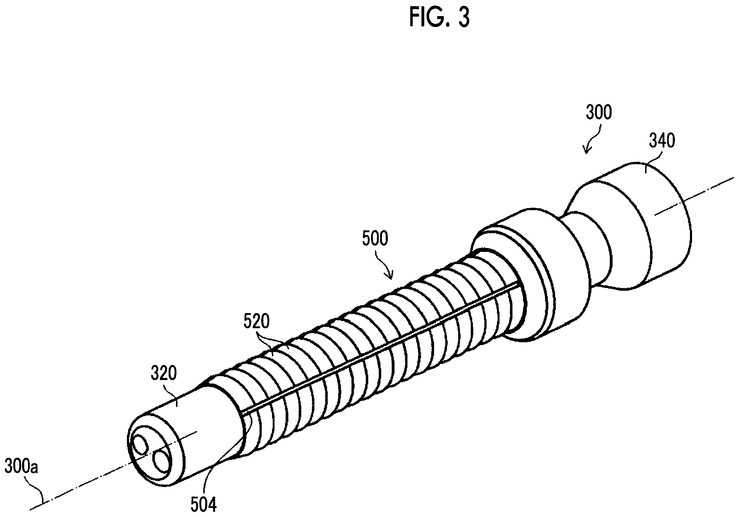

The sheathing tube 500 illustrated in Fig. 1 is formed in a tubular shape, and as illustrated in Fig. 3, is externally fitted (sheathed) to and fixed to an outer peripheral surface of the overtube 300 (a long tubular overtube part 320 to be described below). Although detailed description is omitted, an outer peripheral part of the sheathing tube 500 is provided with a number of lateral grooves 520 running along in a circumferential direction, and four longitudinal grooves 504 running along an axial direction are provided, for example, in four places in the circumferential direction.

Accordingly, in a state where the overtube 300 is inserted into a body wall together with the sheathing tube 500, a number of the lateral grooves 520 of the sheathing tube 500 restrict the forward and backward movement of the sheathing tube 500 with respect to the body wall, and the longitudinal grooves in four places of the sheathing tube 500 restrict the rotation of the sheathing tube 500 in the circumferential direction (around a reference axis 300a) with respect to the body wall. Hence, unintended rotation or forward and backward movement of the overtube 300 fixed to the sheathing tube 500 with respect to the body wall is prevented.

Namely, if the overtube 300 rotates in a direction around the axis with respect to the reference axis 300a that is a central axis (longitudinal axis) of the overtube 300 unintentionally with respect to the body wall or moves forward and backward in the direction (axial direction) of the reference axis 300a in a case where the operation of the treatment tool 200, or the like is performed by inserting the endoscope insertion part 102 and the treatment tool insertion part 202 through the overtube 300 after the overtube 300 (long tubular overtube part 320) is inserted into the body wall, there is a problem that the position of a distal end of the endoscope insertion part 102 may fluctuate and an observation visual field may fluctuate unintentionally. The sheathing tube 500 prevents such unintended fluctuation of the observation visual field.

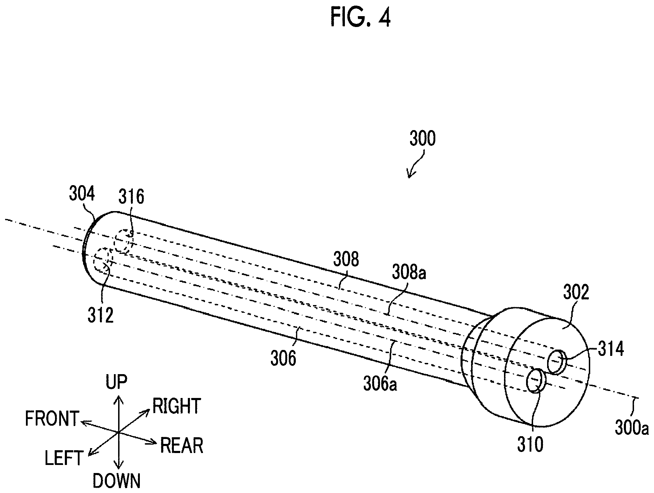

Fig. 4 is an external perspective view illustrating the overtube 300.

As illustrated in this drawing, the overtube 300 has an elongated cylindrical shape as a whole, and has an endoscope insertion passage 306 through which the endoscope insertion part 102 of the endoscope 100 is inserted so as to be movable forward and backward along the reference axis 300a indicating a longitudinal axis thereof, and a treatment tool insertion passage 308 through which the treatment tool insertion part 202 of the treatment tool 200 is inserted so as to be movable forward and backward along the reference axis 300a indicating a longitudinal axis thereof. The endoscope insertion passage 306 is one form of a first insertion passage through which the first insertion part of the first medical instrument is inserted so as to be movable forward and backward, and the treatment tool insertion passage 308 is one form of a second insertion passage through which the second insertion part of the second medical instrument is inserted so as to so as to be movable forward and backward.

Additionally, the endoscope insertion passage 306 and the treatment tool insertion passage 308 are disposed parallel to each other and are disposed parallel to the reference axis 300a. That is, in a case where a central axis of the endoscope insertion passage 306 is referred to as an endoscope insertion axis 306a and a central axis of the treatment tool insertion passage 308 is referred to as a treatment tool insertion axis 308a, the endoscope insertion axis 306a and the treatment tool insertion axis 308a are parallel to each other and are also parallel to the reference axis 300a. The endoscope insertion axes 306a and the treatment tool insertion axes 308a are equivalent to positions of the central axes of the endoscope insertion part 102 and the treatment tool insertion part 202 that are respectively inserted through the endoscope insertion passage 306 and the treatment tool insertion passage 308. Additionally, in the present embodiment, the reference axis 300a, the endoscope insertion axis 306a, and the treatment tool insertion axis 308a are disposed on the same plane. However, a configuration in which the reference axis 300a, the endoscope insertion axis 306a, and the treatment tool insertion axis 308a are disposed on the same plane may not be adopted.

In addition, regarding the position and orientation of a space where the overtube 300 has been disposed, terms called forward, backward, left, right, up, and down are used with the orientation from the proximal end surface 302 in a direction along the reference axis 300a to the distal end surface 304 defined as the forward and with the orientation from the reference axis 300a to the treatment tool insertion axis 308a defined as the right.

The proximal end surface 302 of the overtube 300 is provided with a first proximal end opening 310 that is a proximal end opening that allows the endoscope insertion part 102 to be inserted into the endoscope insertion passage 306 therethrough, and a second proximal end opening 314 that is a proximal end opening that allows the treatment tool insertion part 202 to be inserted into the treatment tool insertion passage 308 therethrough.

The distal end surface 304 of the overtube 300 is provided with a first distal end opening 312 that is a distal end opening that allows the endoscope insertion part 102 to be inserted into the endoscope insertion passage 306 and delivered to the outside therethrough, and a second distal end opening 316 that is a distal end opening that allows the treatment tool insertion part 202 to be inserted into the treatment tool insertion passage 308 and delivered to the outside therethrough.

That is, the endoscope insertion passage 306 that is one form of the first insertion passage allows the first distal end opening 312 and the first proximal end opening 310 to communicate with each other, and the treatment tool insertion passage 308 that is one form of the second insertion passage is provided so as to allow the second distal end opening 316 and the second proximal end opening 314 to communicate with each other.

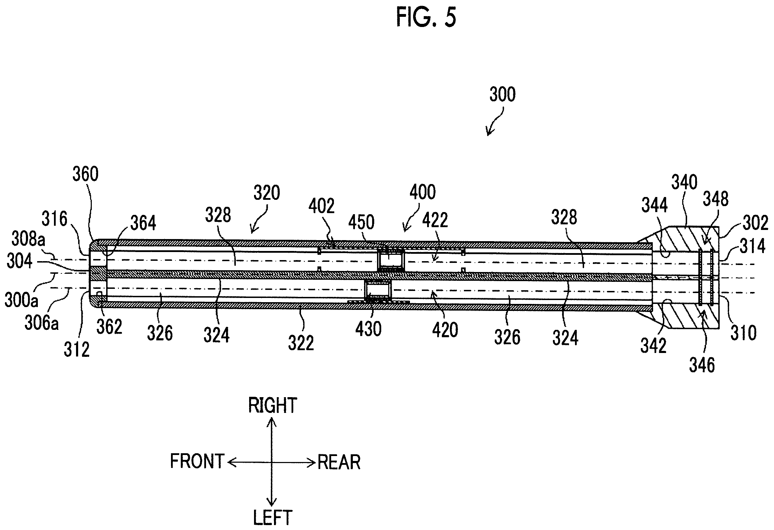

Fig. 5 is a cross-sectional view illustrating the internal structure of the overtube 300, and illustrates a cross section obtained by cutting the overtube 300 in a plane (horizontal plane) that includes the reference axis 300a and is orthogonal to an upward-downward direction.

As illustrated in this drawing, the overtube 300 is constituted by a long tubular overtube part 320 that occupies portions other than a proximal end portion and a distal end portion, a proximal end cap 340 that is attached to a rear end (proximal end) of the overtube 300, and a distal end cap 360 that is attached to a distal end part.

Additionally, the long tubular overtube part 320 is constituted by a long tubular body 322 formed in an elongated cylindrical shape having the reference axis 300a as a central axis (longitudinal axis) using hard resin, metal, or the like, a columnar partition wall member 324 that is housed and disposed inside the long tubular body 322 and extends along the reference axis 300a and that has an endoscope guide groove 326 and a treatment tool guide groove 328 that respectively form portions of the endoscope insertion passage 306 and the treatment tool insertion passage 308, and a slider 400 that is guided by the partition wall member 324 and supported to be movable forward and backward in the forward-backward direction. The details regarding the partition wall member 324 and the slider 400 will be described below.

The proximal end cap 340 is formed in a columnar shape of which the diameter is made larger than the external diameter of the long tubular overtube part 320 (cylindrical body) using hard resins, metals, or the like, and a rear end surface thereof constitutes the proximal end surface 302 of the overtube 300. The proximal end cap 340 is provided with a through-hole 342 and a through-hole 344 that form a portion of the endoscope insertion passage 306 and a portion of the treatment tool insertion passage 308, respectively, and through-holes 342 and 344 respectively communicate with the endoscope guide groove 326 and the treatment tool guide groove 328 of the long tubular overtube part 320. In the proximal end surface 302, an opening of the through-hole 342 is equivalent to the above-described first proximal end opening 310, and an opening of the through-hole 344 is equivalent to the above-described second proximal end opening 314.

Additionally, the through-holes 342 and 344 are provided with valve members 346 and 348. The valve members 346 and 348, for example, open in a case where the endoscope insertion part 102 and the treatment tool insertion part 202 are inserted therethrough and come into close contact with outer peripheral surfaces (side surfaces) of the endoscope insertion part 102 and the treatment tool insertion part 202 without a substantial gap. This secures the airtightness of spaces closer to the distal end side than the valve members 346 and 348, and reduces the leakage or the like of a pneumoperitoneum gas injected into the body cavity to the outside of the body.

The distal end cap 360 is formed of hard resins, metals, or the like, and a front end surface thereof constitutes the distal end surface 304 of the overtube 300. The distal end cap 360 is provided with a through-hole 362 and a through-hole 364 that form a portion of the endoscope insertion passage 306 and a portion of the treatment tool insertion passage 308, respectively, and through-holes 362 and 364 respectively communicate with the endoscope guide groove 326 and the treatment tool guide groove 328 of the long tubular overtube part 320. In the distal end surface 304, an opening of the through-hole 362 is equivalent to the above-described first distal end opening 312, and an opening of the through-hole 364 is equivalent to the second distal end opening 316.

In addition, the long tubular overtube part 320, the proximal end cap 340, and the distal end cap 360 show one form of constituent members that constitutes the overtube body of the overtube 300, and the overtube body is not limited to the above configuration. For example, the long tubular overtube part 320 and the proximal end cap 340 or the long tubular overtube part 320 and the distal end cap 360 may be integrally formed, or may be integrally formed in their entirety.

Additionally, although the distal end surface 304 is simply illustrated in the flat shape in Fig. 5 or the like, detailed shapes thereof will be described below.

The slider 400 in the above-described long tubular overtube part 320 is an interlocking member having a first coupling part coupled to the first insertion part of the first medical instrument inserted through the first insertion passage, and a second coupling part coupled to the second insertion part of the second medical instrument inserted through the second insertion passage, and the partition wall member 324 and the slider 400 constitute a coupling mechanism having the first coupling part and the second coupling part.

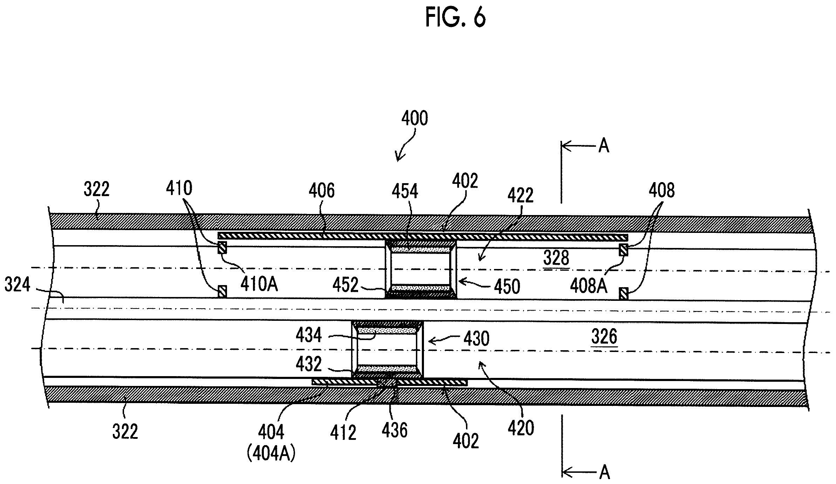

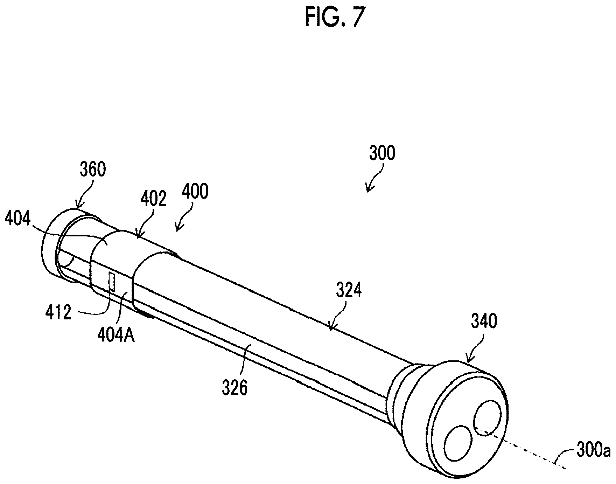

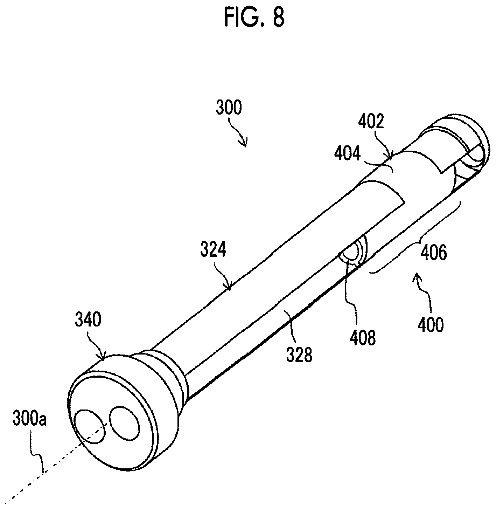

Fig. 6 is a partially enlarged view illustrating a portion, in which the slider 400 is disposed in Fig. 5, in an extracted manner, and Figs. 7 and 8 are perspective views illustrating the overtube 300 from left and right different directions on the proximal end side with the long tubular body 322 in the long tubular overtube part 320 omitted. As illustrated in these drawings, the slider 400 is supported by the columnar partition wall member 324 having the endoscope guide groove 326 and the treatment tool guide groove 328 inside the long tubular body 322.

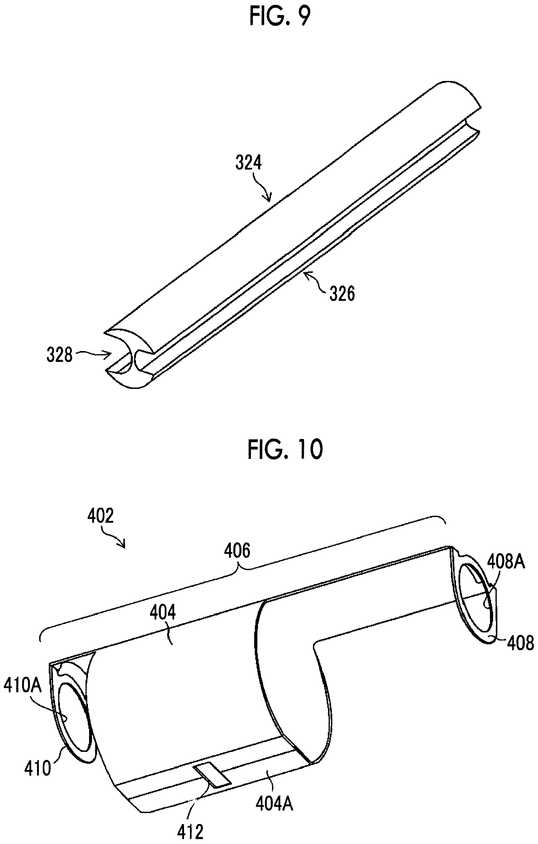

The partition wall member 324 is a solid insulator, has a structure as illustrated in Fig. 9, and extends from the proximal end cap 340 to the distal end cap 360 inside the long tubular body 322.

The endoscope guide groove 326, which forms a portion of the endoscope insertion passage 306 and extends parallel to the reference axis 300a from a proximal end of the partition wall member 324 to a distal end thereof, is formed on a left side of the partition wall member 324. The treatment tool guide groove 328, which forms a portion of the treatment tool insertion passage 308 and extends parallel to the reference axis 300a from the proximal end of the partition wall member 324 to the distal end thereof, is formed on a right side of the partition wall member 324.

That is, the partition wall member 324 has the endoscope guide groove 326 as one form of a first guide groove that constitutes a portion of the first insertion passage, and has the treatment tool guide groove 328 as one form of a second guide groove that constitutes a portion of the second insertion passage. Additionally, the partition wall member 324 forms a partition wall between the first insertion passage and the second insertion passage.

By virtue of the partition wall member 324, the endoscope insertion part 102 and the treatment tool insertion part 202 inserted into the overtube 300 reliably proceeds through the regions of the endoscope insertion passage 306 and the treatment tool insertion passage 308 corresponding thereto, without falling out of the insertion passages, respectively. As a result, the insertion task of the endoscope insertion part 102 with respect to the overtube 300 and the treatment tool insertion part 202 becomes easy.

Additionally, the endoscope insertion part 102 inserted through the endoscope insertion passage 306 and the treatment tool insertion part 202 inserted through the treatment tool insertion passage 308 are prevented from coming into contact with each other inside then overtube 300, and are electrically insulated from each other. For that reason, even in a case where the treatment tool 200 uses electricity, generation of electrical leakage (high-frequency electricity or the like) from the treatment tool 200 to the endoscope 100, electrical noise, or the like can be prevented, and damage or the like to the endoscope 100 can be prevented in advance.

As illustrated in Figs. 6, 7, and 8, the slider 400 is a ring-shaped driving member that is externally fitted to an outer peripheral part of the partition wall member 324 and is movable forward and backward along the reference axis 300a with respect to the partition wall member 324, and has a coupling ring 402 that integrally interlocks components of the slider 400, an endoscope fixing tool 430 disposed as a first fixing tool inside the endoscope guide groove 326 of the partition wall member 324 as illustrated in Fig. 6, and a treatment tool fixing tool 450 disposed as a second fixing tool inside the treatment tool guide groove 328 of the partition wall member 324.

Only the coupling ring 402 is illustrated in Fig. 10. The coupling ring 402 has a tubular ring part 404 that surrounds an outer periphery of the partition wall member 324 in the circumferential direction and that comes into contact with contacts or approaches an outer peripheral surface of the partition wall member 324 in portions other than endoscope guide groove 326 and treatment tool guide groove 328, and an arm part 406 consisting of a portion that faces the treatment tool guide groove 328 of the ring part 404 and a portion that extends in the forward-backward direction from the ring part 404 along a position that faces the treatment tool guide groove 328.

The arm part 406 acts as a second engaging part that is engaged with the treatment tool fixing tool 450 (refer to Fig. 6) that is the second fixing tool, and a proximal end and a distal end of the arm part are respectively provided with a rear restriction end 408 and a front restriction end 410 that are a second restricting part which restricts the forward and backward movement of the treatment tool fixing tool 450 and that are inserted into and disposed in the inside of the treatment tool guide groove 328. Also, the rear restriction end 408 and the front restriction end 410 are respectively provided with openings 408A and 410A through which the treatment tool insertion part 202 is inserted.

Additionally, a first engaging part 404A, which is engaged with the endoscope fixing tool 430 that is the first fixing tool and which extends in a leftward-rightward direction along a plane orthogonal to the forward-backward direction, is formed in a portion that is disposed to face the endoscope guide groove 326 in the ring part 404.

The rotation of the coupling ring 402 in the direction around the axis (a direction around the reference axis 300a) with respect to the partition wall member 324 is restricted by the rear restriction end 408 and the front restriction end 410 that are inserted into and disposed inside the first engaging part 404A and the treatment tool guide groove 328.

Also, the coupling ring 402 is supported so as to be movable forward and backward in the forward-backward direction by the partition wall member 324 within the long tubular overtube part 320, and is supported in a state where the movement of the slider in the upward-downward direction and in the leftward-rightward direction and the rotation of the slider in all directions (direction around three axes including a forward-backward axis, a leftward-rightward axis, and an upward-downward direction) are restricted (a state where the rotation of the slider around at least the reference axis 300a is impossible). Additionally, the coupling ring 402 moves forward and backward within a movable range having a position where the coupling ring 402 (rear restriction end 408) abuts against the proximal end cap 340 as a rear end, and having a position where the coupling ring 402 (front restriction end 410) abuts against the distal end cap 360 as a front end.

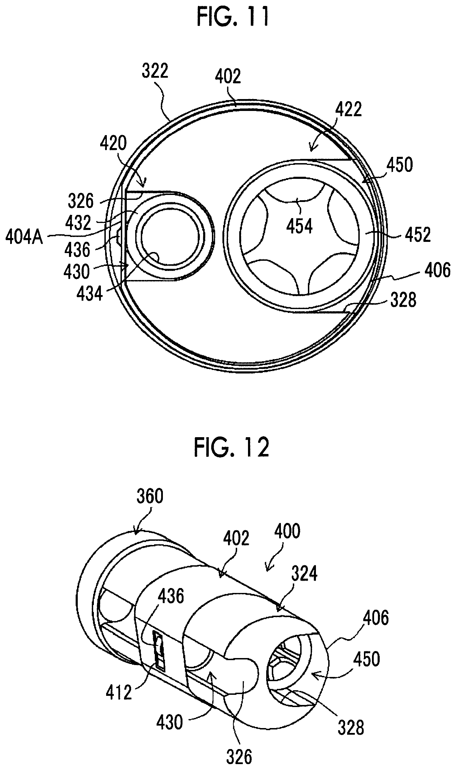

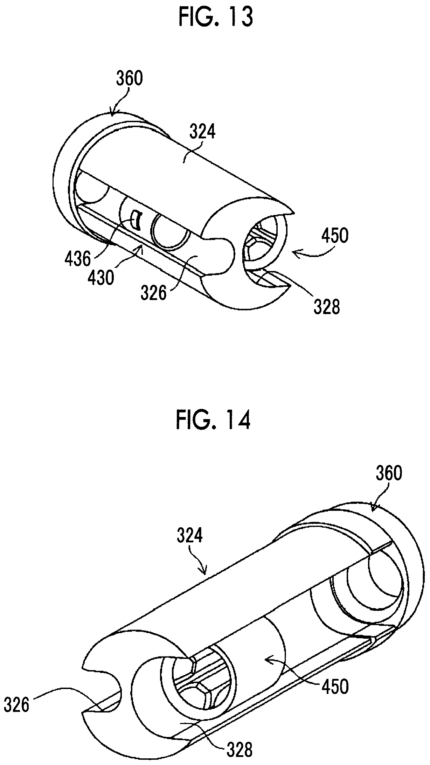

Fig. 11 is a cross-sectional view when viewed from arrow A-A in Fig. 6, and Fig. 12 is a perspective view illustrating the overtube 300 cut by in a plane perpendicular to the reference axis 300a at a position that intersects the arm part 406 closer to the proximal end side than the ring part 404 of the coupling ring 402 in the state of Fig. 7. Fig. 13 is a perspective view illustrating the overtube of Fig. 12 with the coupling ring 402 omitted, and Fig. 14 is a perspective view illustrating the overtube 300 of Fig. 13 from a right side.

As illustrated in Figs. 6 and 11, the slider 400 has a left endoscope coupling part 420 that is coupled to (engaged with) the endoscope insertion part 102 inside the coupling ring 402 and a right treatment tool coupling part 422 that is coupled to (engaged with) the treatment tool insertion part 202.

That is, the slider 400 has an endoscope coupling part 420 as the first coupling part coupled to the first insertion part of the first medical instrument inserted through the first insertion passage, and a treatment tool coupling part 422 as the second coupling part coupled to the second insertion part of the second medical instrument inserted through the second insertion passage.

Specifically, as illustrated in Figs. 6, 11, and 13, the endoscope coupling part 420 provided on a left side of the slider 400 includes the endoscope fixing tool 430 that is disposed inside the endoscope guide groove 326 and that is one form of the first fixing tool that is movable forward and backward in the forward-backward direction along the endoscope insertion passage 306.

The endoscope fixing tool 430 is constituted by a tubular frame 432 that approaches or comes into contact with an inner wall surface of the endoscope guide groove 326, and a tubular pressure-contact member 434 that is fixed inside the frame 432 and formed of an elastic material, such as elastic rubber.

An outer peripheral part of the frame 432 is provided with a protrusion 436 that protrudes in the radial direction from a position in the circumferential direction at a position that faces an opening of the endoscope guide groove 326. As illustrated in Figs. 6, 10, and 12, the protrusion 436 is inserted through an engagement hole 412 that is one form of a first restricting part formed in the first engaging part 404A in the ring part 404 of the coupling ring 402, and is locked to the engagement hole 412 in the forward-backward direction.

According to this, due to the engagement between the protrusion 436 of the endoscope fixing tool 430 and the engagement hole 412 of the coupling ring 402, the endoscope fixing tool 430 and the first engaging part 404A are engaged with each other and the relative forward and backward movement of the endoscope fixing tool 430 in the forward-backward direction with respect to the coupling ring 402 is restricted. Hence, the coupling ring 402 and the endoscope fixing tool 430 integrally move forward and backward in the forward-backward direction.

Additionally, when the endoscope insertion part 102 has been inserted through the endoscope insertion passage 306, the endoscope insertion part 102 is inserted through the inside of the pressure-contact member 434, and the endoscope fixing tool 430 is fixed to the endoscope insertion part 102 by the pressure-contact member 434 being brought into pressure contact with (engaged with) the outer peripheral surface of the endoscope insertion part 102. Then, the central axis of the endoscope insertion part 102 is disposed substantially coaxially with the endoscope insertion axis 306a.

Accordingly, the endoscope insertion part 102 and the slider 400 (coupling ring 402) are coupled to (engaged with) each other in an interlocking manner via the endoscope fixing tool 430, and the slider 400 (coupling ring 402) also integrally moves forward and backward in an interlocking manner with the forward and backward movement of the endoscope insertion part 102 in the forward-backward direction (axial direction).

In addition, since the coupling herein is based on the elastic force of the pressure-contact member 434, the engagement position (the position of the endoscope insertion part 102 where the slider 400 is engaged) of the endoscope insertion part 102 coupled to the slider 400 (coupling ring 402) can be arbitrarily adjusted.

Additionally, the frame 432 of the endoscope fixing tool 430 has a shape such that the movement (rotation) thereof is impossible in the direction around the axis inside the endoscope guide groove 326, and the endoscope fixing tool 430 is allowed only to move forward and backward in the forward-backward direction within the endoscope guide groove 326.

Specifically, as illustrated in Figs. 6, 11, and 14, the treatment tool coupling part 422 provided on a right side of the slider 400 includes the treatment tool fixing tool 450 that is disposed in a range between the rear restriction end 408 and the front restriction end 410 (refer to Fig. 10 or the like) of the arm part 406 of the coupling ring 402 inside the treatment tool guide groove 328, and that is the second fixing tool that is movable forward and backward along the treatment tool guide groove 328.

The treatment tool fixing tool 450 is constituted by a tubular frame 452 that approaches or comes into contact with an inner wall surface of the treatment tool guide groove 328, and a tubular pressure-contact member 454 that is fixed inside the frame 452 and formed of an elastic material, such as elastic rubber. In addition, an inner peripheral surface of the pressure-contact member 454 is formed in a shape such that regularities are repeated in the circumferential direction so as to be appropriately engageable with treatment tool insertion parts 202 having a plurality of types of greatly different diameters.

According to this, when the treatment tool insertion part 202 has been inserted through the treatment tool insertion passage 308, the treatment tool insertion part 202 is inserted through the inside of the pressure-contact member 454, and the treatment tool fixing tool 450 is fixed to the treatment tool insertion part 202 by the pressure-contact member 454 being brought into pressure contact with (engaged with) the outer peripheral surface of the treatment tool insertion part 202. Then, the central axis of the treatment tool insertion part 202 is disposed substantially coaxially with the treatment tool insertion axis 308a.

Accordingly, the treatment tool fixing tool 450 also integrally moves forward and backward in an interlocking manner with the forward and backward movement of the treatment tool insertion part 202 in the forward-backward direction (axial direction). Additionally, the treatment tool fixing tool 450 also rotates inside the treatment tool guide groove 328 in an interlocking manner with the rotation of the treatment tool insertion part 202 around the axis thereof

In addition, since the coupling between the treatment tool insertion part 202 and the treatment tool fixing tool 450 herein is based on the elastic force of the pressure-contact member 454, the engagement position (the position of the treatment tool insertion part 202 where the treatment tool fixing tool 450 is engaged) of the treatment tool insertion part 202 coupled to the treatment tool fixing tool 450 can be arbitrarily adjusted.

Additionally, the rear restriction end 408 in the arm part 406 of the coupling ring 402 is disposed on a rear side of the treatment tool fixing tool 450, and the front restriction end 410 in the arm part 406 is disposed on a front side of the treatment tool fixing tool 450.

Hence, the arm part 406 allows the forward and backward movement of the treatment tool fixing tool 450 in the forward-backward direction with respect to the coupling ring 402 in a range from a position where the treatment tool fixing tool 450 abuts against the rear restriction end 408 to a position where the treatment tool fixing tool 450 abuts against the front restriction end 410, and restricts the treatment tool fixing tool 450 in that range.

Accordingly, the coupling ring 402 has a non-sensing region where either the endoscope fixing tool 430 or the treatment tool fixing tool 450 is not moved forward and backward with the forward and backward movement of the other of the endoscope fixing tool 430 and the treatment tool fixing tool 450.

Meanwhile, in a case where the treatment tool fixing tool 450 moves forward and backward in the forward-backward direction or in a case where the coupling ring 402 moves forward and backward in the forward-backward direction together with the endoscope fixing tool 430, the treatment tool fixing tool 450 abuts against the rear restriction end 408 or the front restriction end 410. In this state, the coupling ring 402 has a sensing region where either the endoscope fixing tool 430 or the treatment tool fixing tool 450 is moved forward and backward movement with respect to the forward and backward movement (the forward and backward movement in a direction in which the treatment tool fixing tool 450 and the rear restriction end 408 or the front restriction end 410 are not spaced apart from each other) of other of the endoscope fixing tool 430 and the treatment tool fixing tool 450.

Due to the configuration of the above slider 400, the slider 400 has the non-sensing region where either the endoscope insertion part 102 inserted through the endoscope insertion passage 306 of the overtube 300 and coupled to the endoscope fixing tool 430 or the treatment tool insertion part 202 inserted through the treatment tool insertion passage 308 and coupled to the treatment tool fixing tool 450 does not move forward and backward without interlocking with the forward and backward movement of the other in the forward-backward direction (axial direction) and the sensing region where either the endoscope insertion part 102 or the treatment tool insertion part 202 moves forward and backward in an interlocking manner with the forward and backward movement of the other. That is, the endoscope insertion part 102 is adapted to interlock with the forward and backward movement of the treatment tool insertion part 202 in the axial direction with play by the slider 400.

The working of the overtube 300 configured as described above will be described together with the operation in a case where the treatment of a diseased site within a patient's body cavity is performed using the endoscopic surgical device 10.

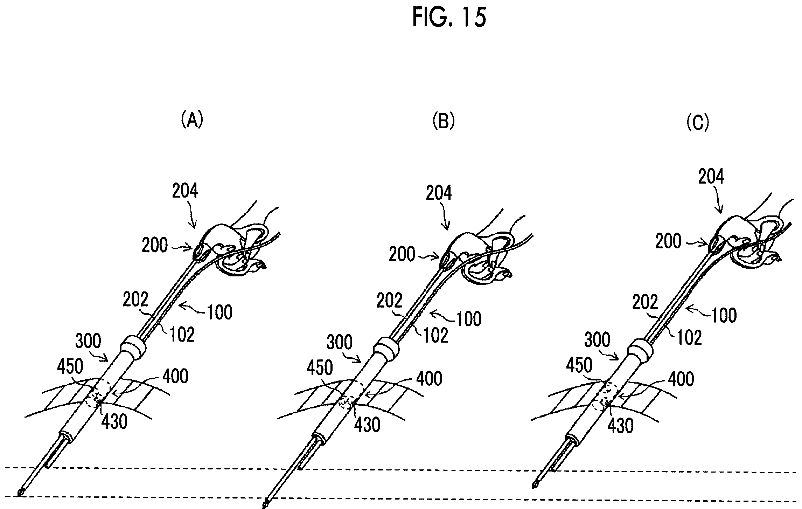

First, as illustrated in portion (A) of Fig. 15, after the overtube 300 is inserted into a patient's body wall and a pneumoperitoneum gas is injected into a body cavity, the endoscope 100 (endoscope insertion part 102) and the treatment tool 200 (treatment tool insertion part 202) are respectively inserted into the endoscope insertion passage 306 and the treatment tool insertion passage 308 of the overtube 300, and the endoscope insertion part 102 and the treatment tool insertion part 202 are mounted on the overtube 300.

In this case, the endoscope insertion part 102 is reliably guided to a position, where the endoscope fixing tool 430 of the slider 400 is inserted, by the endoscope guide groove 326 of the partition wall member 324, and is coupled to the endoscope fixing tool 430.

Similarly, the treatment tool insertion part 202 is guided reliably to a position, where the treatment tool fixing tool 450 of the slider 400 is inserted, by the treatment tool guide groove 328 of the partition wall member 324, and is coupled to the treatment tool fixing tool 450.

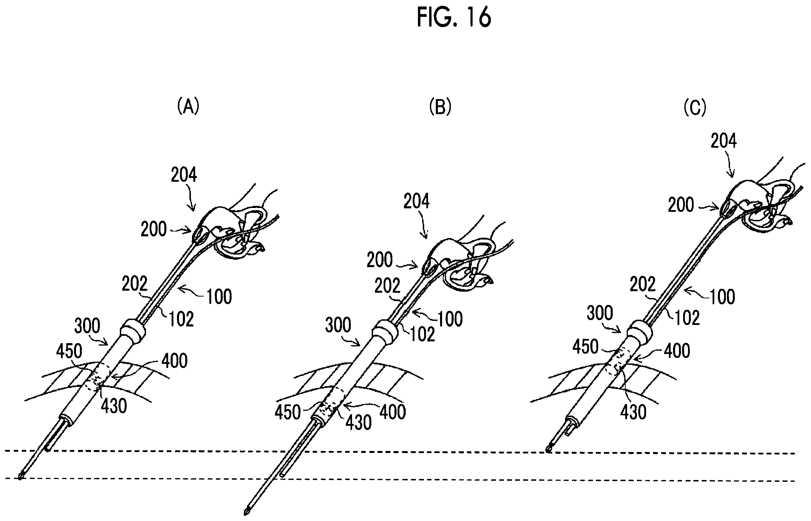

In addition, although the sheathing tube 500 is not illustrated in Fig. 15, and Fig. 16 illustrated therebelow, the sheathing tube 500 is fitted to the overtube 300 as illustrated in Fig. 3. However, it is also possible to use the overtube 300 without fitting the sheathing tube 500 thereto.

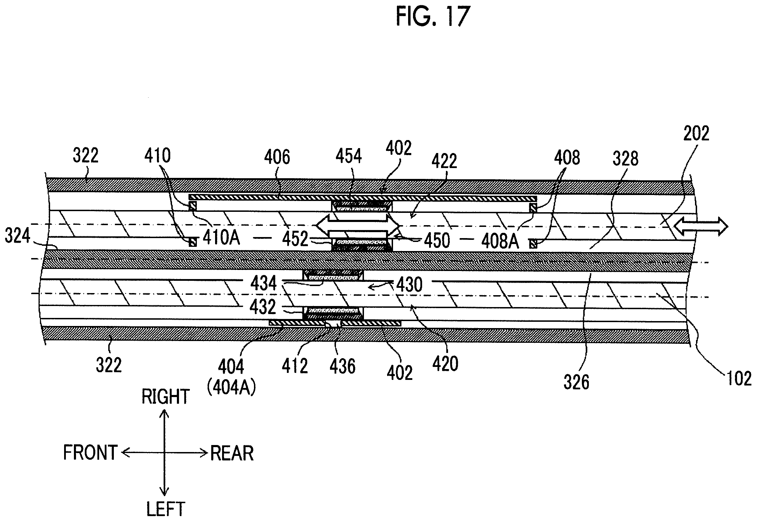

It is assumed that the state of portion (A) of Fig. 15 is a state illustrated in Fig. 17. Fig. 17 is a cross-sectional view illustrating the state of the slider 400 coupled to the endoscope insertion part 102 and the treatment tool insertion part 202, and illustrating a state where the treatment tool fixing tool 450 reaches neither a front end nor a rear end of a movable range thereof with respect to the coupling ring 402 (arm part 406). That is, a state where the treatment tool fixing tool 450 reaches neither the rear restriction end 408 nor the front restriction end 410 is illustrated.

In this case, if the operator minutely moves the treatment tool insertion part 202 forward with his/her hand that is gripping the operating part 204 of the treatment tool 200, only the treatment tool fixing tool 450 moves forward within the movable range thereof with respect to the coupling ring 402, and the coupling ring 402 does not move with respect to the overtube 300 (long tubular overtube part 320).

For that reason, with respect to the forward movement of the treatment tool insertion part 202 until the treatment tool fixing tool 450 reaches the front end (front restriction end 410) of the movable range thereof with respect to the coupling ring 402, as illustrated in portion (B) of Fig. 15, only the treatment tool insertion part 202 moves forward in a state where the endoscope insertion part 102 is stationary. That is, the slider 400 has the non-sensing region where the endoscope insertion part 102 does not interlock with the forward and backward movement of the treatment tool insertion part 202, and the forward movement operation of the treatment tool 200 at this time becomes a forward and backward movement operation of the slider 400 in the non-sensing region.

Similarly, in the state illustrated in Fig. 17, if the operator minutely moves the treatment tool insertion part 202 backward with his/her hand that is gripping the operating part 204 of the treatment tool 200, only the treatment tool fixing tool 450 moves backward within the movable range thereof with respect to the coupling ring 402, and the coupling ring 402 does not move with respect to the overtube 300 (long tubular overtube part 320).

For that reason, with respect to the backward movement of the treatment tool insertion part 202 until the treatment tool fixing tool 450 reaches the rear end (rear restriction end 408) of the movable range thereof with respect to the coupling ring 402, as illustrated in portion (C) of Fig. 15, only the treatment tool insertion part 202 moves backward in a state where the endoscope insertion part 102 is stationary. That is, the backward movement operation of the treatment tool 200 at this time becomes a backward movement operation of the slider 400 in the non-sensing region.

Hence, since the endoscope 100 does not move forward and backward with respect to the minute forward and backward movement operation of the treatment tool 200, that is, the forward and backward movement operation thereof in the non-sensing region, the range of an observation site, such as a distal end site of the treatment tool 200 or a body cavity inner site, to be displayed on the monitor 112 as an endoscopic image does not vary, and the size of an image of the observation site can be prevented from fluctuating according to minute displacement of the treatment tool 200. Accordingly, a sense of perspective can be suitably maintained, and a stable endoscopic image can be provided.

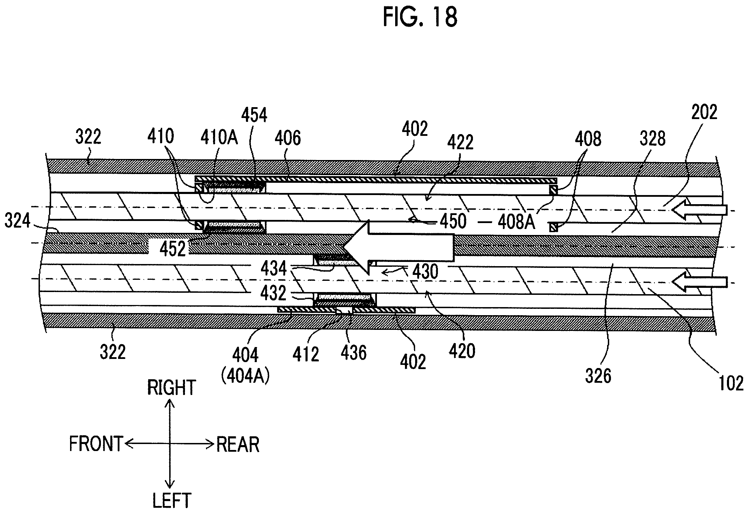

Meanwhile, if the operator greatly moves the treatment tool insertion part 202 forward with his/her hand that is gripping the operating part 204 of the treatment tool 200 in the state illustrated in Fig. 17, a state where the treatment tool fixing tool 450 reaches the front end (front restriction end 410) of the movable range thereof with respect to the coupling ring 402 as illustrated in Fig. 18 is brought out after the forward movement of the treatment tool fixing tool 450 of the slider 400 in the non-sensing region until it abuts against the front end (front restriction end 410) of the movable range. Then, in a case where the treatment tool insertion part 202 further moves forward, the treatment tool fixing tool 450 and the coupling ring 402 move forward with respect to the long tubular overtube part 320 together with the treatment tool insertion part 202. Then, the endoscope fixing tool 430 moves forward together with the coupling ring 402, and the endoscope insertion part 102 moves forward together with the endoscope fixing tool 430. Accordingly, the endoscope insertion part 102 moves forward in an interlocking manner with the treatment tool insertion part 202.

For that reason, with respect to the forward movement of the treatment tool insertion part 202 after the treatment tool fixing tool 450 reaches the front end (front restriction end 410) of the movable range thereof with respect to the coupling ring 402, the endoscope insertion part 102 moves forward in an interlocking manner with the treatment tool insertion part 202 as illustrated in portion (B) of Fig. 16, compared to the state of portion (A) of Fig. 16 illustrating the same state as portion (A) of Fig. 15. That is, the slider 400 has the sensing region where the endoscope insertion part 102 interlocks with the forward and backward movement of the treatment tool insertion part 202, and the forward movement operation of the treatment tool 200 at this time becomes a forward movement operation of the slider 400 in the sensing region.

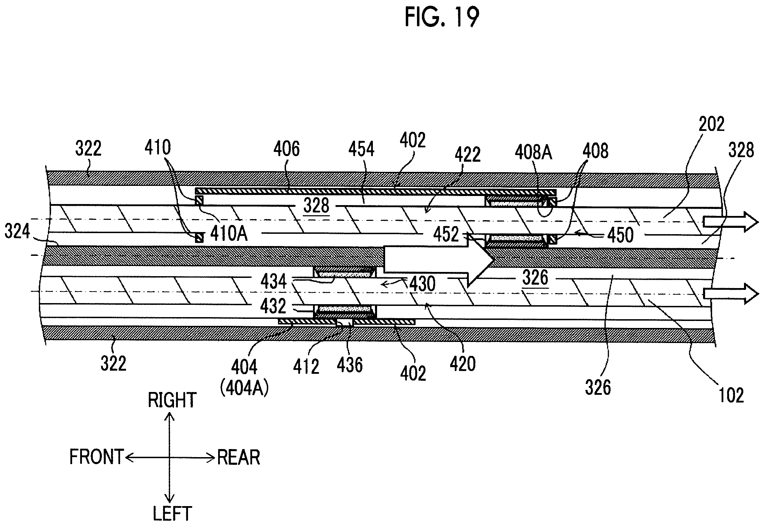

Similarly, if the operator greatly moves the treatment tool insertion part 202 backward with his/her hand that is gripping the operating part 204 of the treatment tool 200 in the state illustrated in Fig. 17, a state where the treatment tool fixing tool 450 reaches the rear end (rear restriction end 408) of the movable range thereof with respect to the coupling ring 402 as illustrated in Fig. 19 is brought out after the backward movement of the treatment tool fixing tool 450 of the slider 400 in the non-sensing region until it abuts against the rear end (rear restriction end 408) of the movable range. Then, in a case where the treatment tool insertion part 202 further moves backward, the treatment tool fixing tool 450 and the coupling ring 402 moves backward with respect to the long tubular overtube part 320 together with the treatment tool insertion part 202. Then, the endoscope fixing tool 430 moves backward together with the coupling ring 402, and the endoscope insertion part 102 moves backward together with the endoscope fixing tool 430. Accordingly, the endoscope insertion part 102 moves backward in an interlocking manner with the treatment tool insertion part 202.

For that reason, with respect to the backward movement of the treatment tool insertion part 202 after the treatment tool fixing tool 450 reaches the rear end (rear restriction end 408) of the movable range thereof with respect to the coupling ring 402, as illustrated in portion (C) of Fig. 16, the endoscope insertion part 102 moves backward in an interlocking manner with the treatment tool insertion part 202. That is, the backward movement operation of the treatment tool 200 at this time becomes a backward movement operation of the slider 400 in the sensing region.

Hence, since the endoscope 100 moves forward and backward with respect to a large forward and backward movement operation of the treatment tool 200, that is, the forward and backward movement operation thereof in the sensing region, the range of an observation site that appears in an endoscopic image to be displayed on the monitor 112 is continuously changed so as to follow the forward and backward movement of the treatment tool 200. Since the size of images of observation sites other than the distal end site of the treatment tool 200 that appears in the endoscopic image according to the operation of the treatment tool 200, and the size of the range of the observation site varies, the operator can simply obtain a desired image.

As described above, in a case where the displacement of the treatment tool insertion part 202 in the axial direction is large (in a case where a large amplitude of forward and backward movement operation has been performed) when an operator has moved the treatment tool insertion part 202 forward and backward in the axial direction, the endoscope insertion part 102 also moves forward, backward, up, down, right, and left in an interlocking manner. Thus, the visual field, orientation, and the like of the endoscope 100 can be changed as intended by an operator. Additionally, the visual field is always given to pick up an image of the distal end site of the treatment tool 200 and consequently, an image that is optimal for treatment is automatically provided. In a case where it is desired to check sites other than a site to be treated, the checking can be performed by moving the treatment tool insertion part 202, and an operator can perform operations as desired. Hence, an assistant (endoscopic technician) who operates the endoscope 100 apart from the operator can be made unnecessary, and a troublesome condition in which the operator should instruct an assistant about the visual field, orientation, and the like of the endoscope 100 serially can be eliminated.

Additionally, in a case where the displacement of the treatment tool insertion part 202 in the axial direction is small (in a case where a small amplitude of forward and backward movement has been performed), the endoscope insertion part 102 does not interlock. Therefore, an endoscopic image can be prevented from fluctuating unnecessarily, a sense of perspective can be suitably maintained, and a stable endoscopic image can be provided.

As described above, in the overtube 300 of the above embodiment, the insertion passage through which the endoscope 100 (endoscope insertion part 102) is inserted is used as the first insertion passage, and the insertion passage through which the treatment tool 200 (treatment tool insertion part 202) is inserted is used as the second insertion passage. The invention can be applied to an overtube including the first insertion passage through which the first insertion part of the first medical instrument of the first medical instrument and the second medical instrument that are arbitrary types of two medical instruments is inserted, and the second insertion passage through which the second insertion passage of the second medical instrument is inserted.

Additionally, in the above embodiment, the configuration of the slider 400 that is an interlocking member consisting of the first coupling part that is movable forward and backward inside the overtube 300 and is coupled to the first insertion part of the first medical instrument inserted through the first insertion passage, and the second coupling part that is coupled to the second insertion part of the second medical instrument inserted through the second insertion passage, or the configuration of the coupling mechanism consisting of the partition wall member 324 and the slider 400 is an example, or those having other configurations may be adopted.

Additionally, although the slider 400 of the above embodiment has the non-sensing region, the invention can also be applied to a case where the slider 400 has no non-sensing region and has only the sensing region. Moreover, in the invention, the overtube 300 may not have the interlocking member (coupling mechanism), such as the slider 400, and may have simply the first insertion passage and the second insertion passage through which two medical instruments are inserted.

Additionally, in the overtube 300 of the above embodiment, the endoscope insertion passage 306 that is the first insertion passage, and the treatment tool insertion passage 308 that is the second insertion passage are disposed parallel to each other. However, the invention is not limited to this, and can also be applied to overtubes in which the first insertion passage and the second insertion passage obliquely intersect each other.

That is, in the above embodiment, if the endoscope insertion axis 306a that is the central axis of the endoscope insertion passage 306 and the treatment tool insertion axis 308a that is the central axis of the treatment tool insertion passage 308 are parallel to the reference axis 300a, and the endoscope insertion axis 306a and the treatment tool insertion axis 308a are parallel to each other, these axes may not be necessarily parallel to each other.

For example, the invention can also be applied to a form in which the treatment tool insertion passage 308 that is the second insertion passage is disposed parallel to the reference axis 300a as in the above embodiment, and the endoscope insertion passage 306 that is the first insertion passage is disposed to obliquely intersect the reference axis 300a. The overtube of this form will be specifically described as a modification example of the overtube 300 of the above embodiment. In the following embodiment shown as the modification example, constituent elements having functions that are the same as or similar to those of the constituent elements of the above embodiment will be designated by the same reference signs.

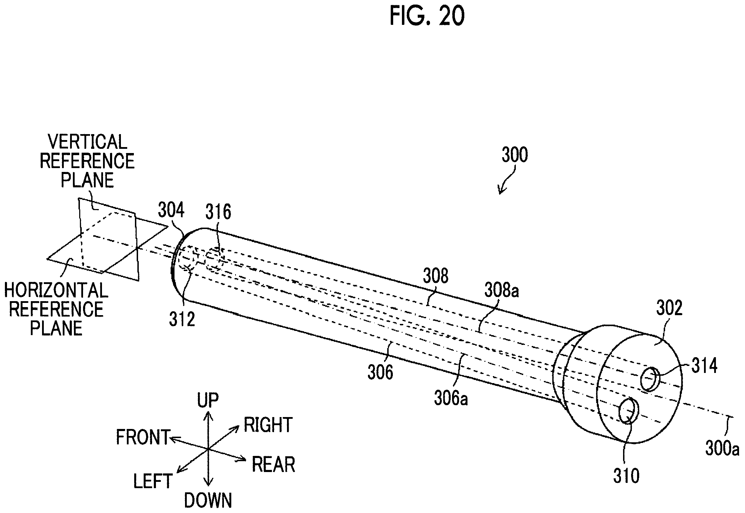

Fig. 20 is an external perspective view of the overtube 300 that is the modification example.

In this drawing, the treatment tool insertion axis 308a of the treatment tool insertion passage 308 is disposed parallel to the reference axis 300a of the overtube 300, and the endoscope insertion axis 306a of the endoscope insertion passage 306 obliquely intersects the reference axis 300a.

That is, in a case where a plane along the upward-downward direction including the reference axis 300a is referred to as a vertical reference plane and a plane along the leftward-rightward direction including the reference axis 300a is referred to as a horizontal reference plane, the treatment tool insertion axis 308a is parallel to both the horizontal reference plane and the vertical reference plane.

Meanwhile, the endoscope insertion axis 306a is parallel to the vertical reference plane and is not parallel to the horizontal reference plane, and is obliquely inclined with respect to the horizontal reference plane. Also, the endoscope insertion axis 306a is inclined from a rear lower side toward a front upper side, and for example, intersects the horizontal reference plane at a substantially intermediate position of the overtube 300 in the forward-backward direction.

In a case where the overtube 300 illustrated in this Fig. 20 is configured, the endoscope guide groove 326 in the long tubular overtube part 320, the through-hole 342 in the proximal end cap 340, and the through-hole 362 in the distal end cap 360 are obliquely formed with respect to the horizontal reference plane along the endoscope insertion axis 306a.

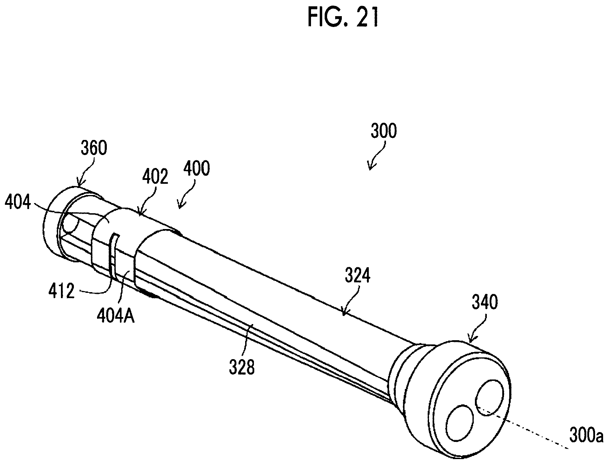

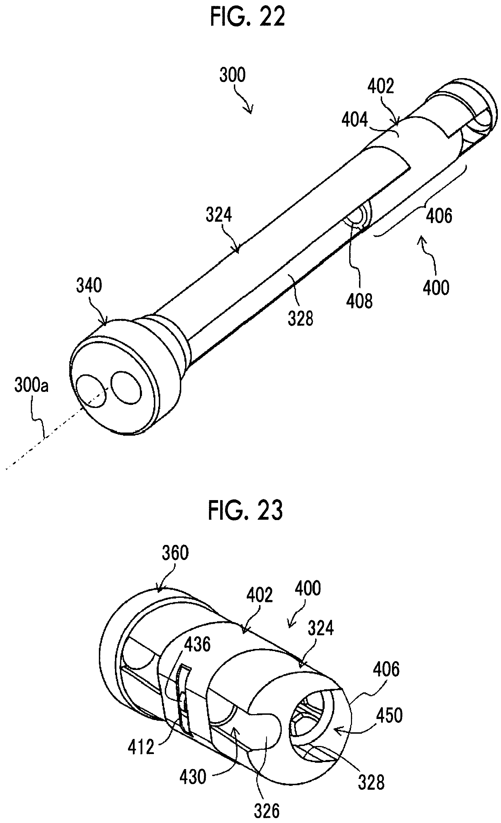

Figs. 21 and 22 are perspective views illustrating the partition wall member 324 and the slider 400 in the long tubular overtube part 320 in a case where the overtube 300 of Fig. 20 is configured. As illustrated in Fig. 22, the treatment tool guide groove 328 of the partition wall member 324 is formed along the treatment tool insertion axis 308a parallel to the reference axis 300a as in the above embodiment.

Meanwhile, as illustrated in Fig. 21, the endoscope guide groove 326 of the partition wall member 324 is not parallel to the reference axis 300a, and is formed along the endoscope insertion axis 306a that is oblique with respect to the horizontal reference plane.

Additionally, since the endoscope fixing tool 430 disposed inside the endoscope guide groove 326 moves also in the upward-downward direction with respect to the partition wall member 324 and the coupling ring 402 together with the forward and backward movement in the forward-backward direction, the protrusion 436 formed on the outer peripheral part of the endoscope fixing tool 430 also moves in the upward-downward direction with respect to the coupling ring 402 according to the position of the endoscope fixing tool 430 in the forward-backward direction.

Thus, the engagement hole 412 formed in the flat first engaging part 404A of the coupling ring 402 is formed as an elongated hole that extends in the circumferential direction (upward-downward direction) beyond the range of the first engaging part 404A as illustrated in the enlarged view of Fig. 23 so as to be engaged with of the protrusion 436 at an arbitrary position of a movement range of the protrusion 436 in the upward-downward direction.

Additionally, since the first engaging part 404A of the coupling ring 402 is the plane orthogonal to the leftward-rightward direction, the distance between the outer peripheral surface of the endoscope fixing tool 430 (not illustrated) and the first engaging part 404A is uniformly maintained irrespective of the movement of the endoscope fixing tool 430 in the upward-downward direction with respect to the coupling ring 402. For that reason, the amount of protrusion of the protrusion 436 can be reduced, and the diameter of the long tubular overtube part 320 can be reduced.

Meanwhile, in a case where the endoscope guide groove 326 is obliquely formed, the opening of the endoscope guide groove 326 deviates from a position that faces the first engaging part 404A. Therefore, the range of the partition wall member 324 through which the first engaging part 404A passes due to the movement of the coupling ring 402 in the forward-backward direction is cut out along a flat surface so as not to interfere with the first engaging part 404A.

According to such an overtube 300, the distal end of the endoscope insertion part 102 and the distal end of the treatment tool insertion part 202 through which the overtube 300 is inserted can be spaced apart from each other even in a case where the spacing between the endoscope insertion passage 306 and the treatment tool insertion passage 308 in the overtube 300 is narrowed for reduction in diameter. Therefore, there is an advantage that the state of the distal end (treatment part 206) of the treatment tool 200 with an endoscope 100 is easily observed.

Next, the inner needle 600 used by being mounted on the overtube 300 in a case where a body wall is punctured with the overtube 300 will be described. In addition, since the inner needle used for the overtube 300 of Figs. 1 to 19 where the endoscope insertion passage 306 and the treatment tool insertion passage 308 are disposed parallel to each other, and the inner needle used for the overtube 300 illustrated in Figs. 20 to 23 where the endoscope insertion passage 306 and the treatment tool insertion passage 308 are disposed to obliquely intersect each other does not have a large constitutional difference in a characterizing portion, a detailed configuration detailed regarding the former inner needle will be described.

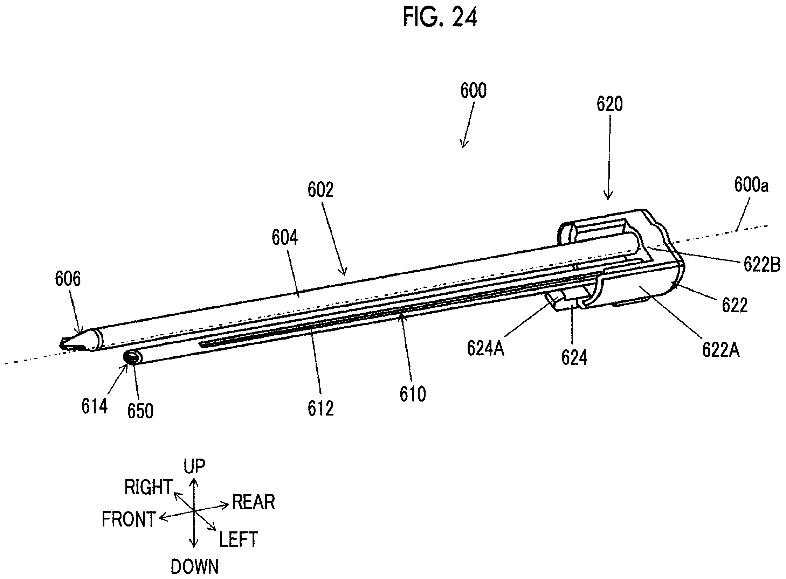

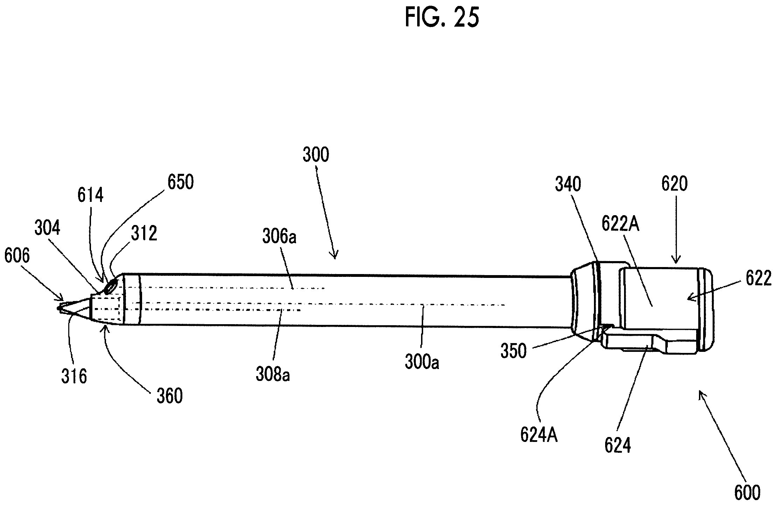

Fig. 24 is a perspective view illustrating the inner needle 600, which is mounted on the overtube 300, from an upper left front side, and Fig. 25 is a perspective view illustrating the overtube 300, on which the inner needle 600 is mounted, from a lower left side.

In addition, the sheathing tube 500 illustrated in the Fig. 1 or the like will be omitted.

Additionally, the distal end surface 304 (the distal end surface 304 in the distal end cap 360) of the overtube 300 is flat also in the drawings and the detailed shape thereof is omitted in the above description of the overtube 300. However, in the following description, a shape corresponding to the embodiment is illustrated as in Fig. 25.

Additionally, as illustrated in Fig. 25, an axis of the inner needle 600 disposed coaxially with the reference axis 300a of the overtube 300 in a state where the inner needle 600 is mounted on the overtube 300 is referred to as a reference axis 600a.

Additionally, front and rear, left and right, and upper and lower relationships of the inner needle 600 shall follow the front and rear, left and right, and upper and lower relationships of the overtube 300 in a state where the inner tube is mounted on the overtube 300 as illustrated in Fig. 25.

As illustrated in these drawings, the inner needle 600 has two needle parts 602 and 610 that extend parallel to the forward-backward direction and are sequentially installed left and right, that is, a right long needle part 602 that is inserted into the treatment tool insertion passage 308 of the overtube 300 and a left short needle part 610 that is inserted into the endoscope insertion passage 306 of the overtube 300 and is shorter than the long needle part 602. Additionally, the inner needle 600 has a head part 620 that integrally and fixedly holds the long needle part 602 and the short needle part 610 on proximal end sides thereof.

Here, in a case where the needle part of the inner needle 600 inserted through the first insertion passage of the overtube 300 is referred to as the first needle part and the needle part of the inner needle 600 inserted through the second insertion passage of the overtube 300 is referred to as the second needle part, the long needle part 602 is equivalent to the second needle part, and the short needle part 610 is equivalent to the first needle part.

Additionally, the head part 620 is equivalent to a positioning part that defines the position of the distal end part of the first needle part with respect to the first distal end opening 312 and the position of the distal end part of the second needle part with respect to the second distal end opening 316 when the head part 620 is in a state (hereinafter referred to as a "mounting state between the overtube 300 and the inner needle 600") where the inner needle 600 is mounted on the overtube 300 as illustrated in Fig. 25, that is, when the head part 620 is in a state where the overtube 300 and the inner needle 600 are combined together.

The long needle part 602 has a shaft part 604 that extends in a rod shape (tubular shape) in parallel with the reference axis 600a from the head part 620 to the distal end side as illustrated in Fig. 24.

The shaft part 604 is disposed at a position where a central axis of the shaft part 604 becomes substantially coaxial with the treatment tool insertion axis 308a of the overtube 300, in the mounting state between the overtube 300 and the inner needle 600 as illustrated in Fig. 25.

Additionally, the shaft part 604 has a thickness such that that the shaft part is insertable through the treatment tool insertion passage 308. Also, the shaft part 604 has a length such that a distal end of the shaft part 604 substantially coincides with an opening surface of the second distal end opening 316 in the distal end surface 304 (distal end cap 360) of the overtube 300 in a state where the long needle part 602 is inserted into the treatment tool insertion passage 308 up to a predetermined position, in the mounting state between the overtube 300 and the inner needle 600.

A distal end part 606 having a tapered shape (a conical shape) that has an inclined surface tapered toward the distal end is provided as a second distal end part at the distal end of the shaft part 604. Also, in the mounting state between the overtube 300 and the inner needle 600 as illustrated in Fig. 25, the distal end part 606 is positioned at a position where the distal end part protrudes from the second distal end opening 316.

In addition, in the long needle part 602, a hollow cylindrical body may be used as the shaft part 604, a distal end opening of the shaft part may be blocked by the distal end part 606 formed of a transparent member, and an observation device, such as an endoscope, may be inserted into the inside of the shaft part 604 from the proximal end opening, so that a frontal state can be observed from the distal end part 606 of the long needle part 602.

The details regarding the shape or the like of the distal end part 606 of the long needle part 602 will be described below.

The short needle part 610 has a shaft part 612 that extends in a rod shape in parallel with the reference axis 600a from the head part 620 to the distal end side as illustrated in Fig. 24.

The shaft part 612 is disposed at a position where the central axis of the shaft part 612 becomes substantially coaxial with the endoscope insertion axis 306a of the overtube 300, in the mounting state between the overtube 300 and the inner needle 600 as illustrated in Fig. 25.

Additionally, the shaft part 612 has a thickness such that that the shaft part is insertable through the endoscope insertion passage 306. Also, the shaft part 612 has a length such that a distal end of the shaft part 612 substantially coincides with an opening surface of the first distal end opening 312 in the distal end surface 304 (distal end cap 360) of the overtube 300 in a state where the short needle part 610 is inserted into the endoscope insertion passage 306 up to a predetermined position, in the mounting state between the overtube 300 and the inner needle 600.

In addition, in the present embodiment, the internal diameter of the endoscope insertion passage 306 is smaller than the internal diameter of the treatment tool insertion passage 308 (the internal diameter of the second insertion part is larger than the internal diameter of the first insertion passage). Therefore, the short needle part 610 is thinner than the long needle part 602 (the external diameter of the long needle part 602 is larger than the external diameter of the short needle part 610).

A distal end part 614 having a cutting blade part 650 is provided as a first distal end part at the distal end of the shaft part 612, and as illustrated in Fig. 25, the distal end part 614 is positioned at a position where the distal end part protrudes from the first distal end opening 312, in the mounting state between the overtube 300 and the inner needle 600 as illustrated in Fig. 25.

In addition, a distal end surface 304 of the distal end of the shaft part 612 can also be used as a constituent element of the first distal end part.

The details regarding the shape or the like of the distal end part 614 of the short needle part 610 will be described below.

As illustrated in Fig. 24, the head part 620 has a head part body 622 consisting of a side wall part 622A along a range of a substantially lower half of a cylindrical surface having the reference axis 600a as a central axis, and a substantially semicircular plate-shaped bottom part 622B at a proximal end of the side wall part 622A.

A proximal end of the long needle part 602 and a proximal end of the short needle part 610 are fixedly provided at the bottom part 622B.

A long plate-shaped locking lever 624 that extends in the forward-backward direction is provided at a position below the reference axis 600a in the side wall part 622A. The locking lever 624 is supported such that a distal end part and a proximal end part thereof are rockable in the upward-downward direction (in the radial direction with respect to the reference axis 600a) with the vicinity of the center thereof in the forward-backward direction as a fulcrum. In addition, a distal end part of the locking lever 624, is biased upward (radially inward with respect to the reference axis 600a) by biasing means and a proximal end side thereof is biased downward by the biasing means.

A locking claw 624A is provided to protrude from an upper surface side (reference axis 600a side) of the distal end part of the locking lever 624, and the locking claw 624A has a shape such that the locking claw is fitted to a locking hole 350 (refer to Fig. 25) provided in the proximal end cap 340 of the overtube 300.

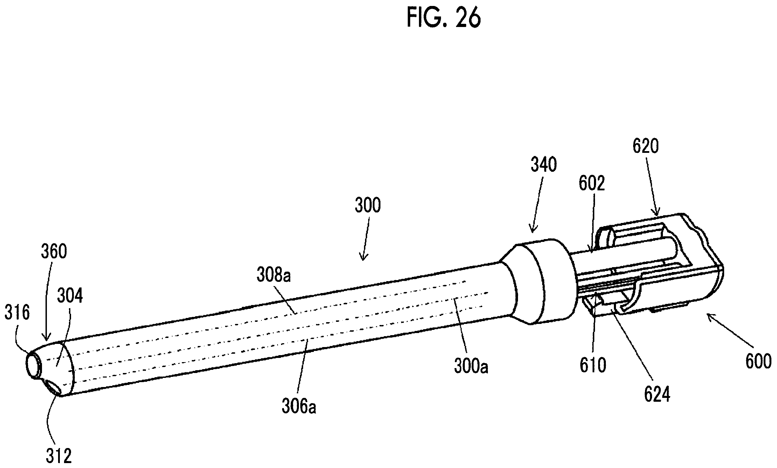

According to the inner needle 600 configured as above, if the long needle part 602 and the short needle part 610 of the inner needle 600 are respectively inserted into the treatment tool insertion passage 308 and the endoscope insertion passage 306 from the second proximal end opening 314 and the first proximal end opening 310, respectively, of the overtube 300, the head part 620 of the inner needle 600 approaches the proximal end cap 340 of the overtube 300 as illustrated in Fig. 26.

Then, in a case where the inner needle 600 is further inserted, as illustrated in Fig. 25, a front end of the side wall part 622A of the head part body 622 abuts against the proximal end surface 302 of the proximal end cap 340 of the overtube 300, and the locking claw 624A of the locking lever 624 is fitted to the locking hole 350 of the proximal end cap 340. This brings about a state where the inner needle 600 is mounted on the overtube 300 and a state where the overtube 300 and the inner needle 600 are combined together.

In this case, the distal end part 606 of the long needle part 602 of the inner needle 600 and the distal end part 614 of the short needle part 610 are positioned at positions where these distal end parts protrude from the distal end surface 304 of the overtube 300.

In a case where endoscopic surgery is performed, in the mounting state between the overtube 300 and the inner needle 600, the distal end part 606 of the long needle part 602 protruding to a forefront side punctures a body wall from a skin-incised part (incised wound) formed in the body wall, and the overtube 300 is inserted into the body cavity.

Meanwhile, if a proximal end part of the locking lever 624 is pressed in the mounting state between the overtube 300 and the inner needle 600, the locking claw 624A can be removed from the locking hole 350 of the proximal end cap 340, and if the inner needle 600 is pulled out to the hand side in that state, the inner needle 600 can be detached from the overtube 300.

Subsequently, the shapes of the distal end surface 304 (distal end cap 360) of the overtube 300, the distal end part 606 of the long needle part 602 of the inner needle 600, and the distal end part 614 of the short needle part 610 will be described.

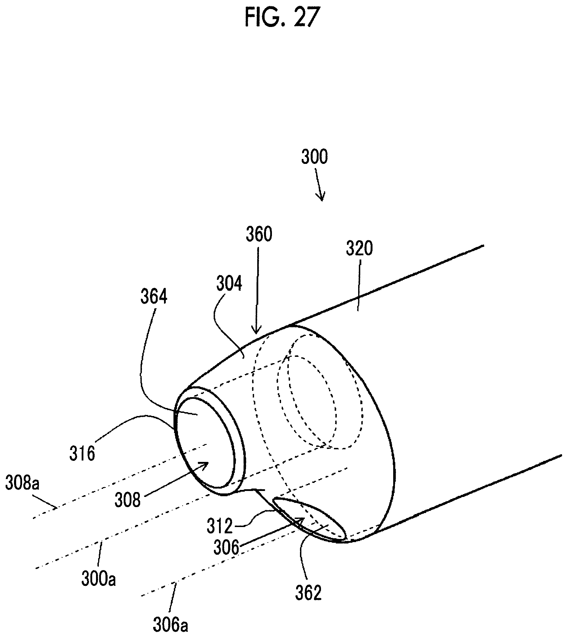

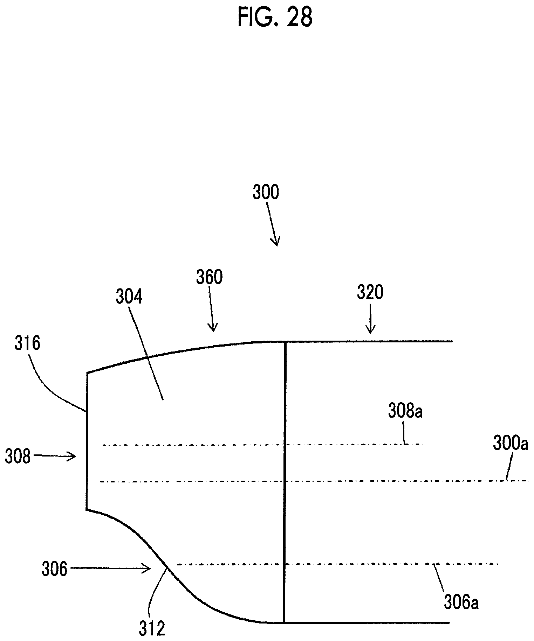

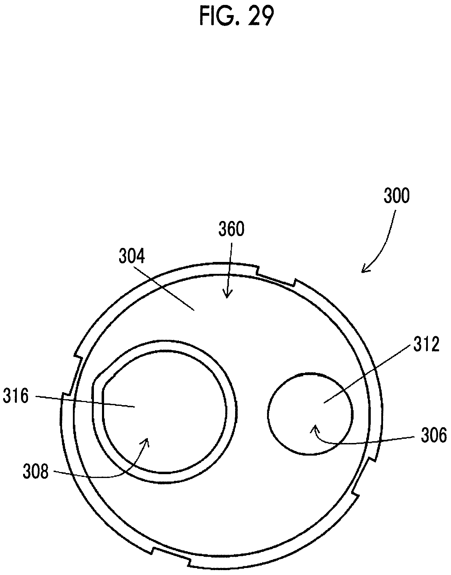

Fig. 27 is a perspective view illustrating a distal end portion (a peripheral part of the distal end cap 360) of the overtube 300 in a state (hereinafter referred to as a "non-mounting state of the inner needle 600") where the inner needle 600 is not mounted on the overtube 300, Fig. 28 is a plan view illustrating the distal end portion of the overtube 300 from an upper side in the non-mounting state of the inner needle 600, and Fig. 29 is a front view illustrating the distal end portion of the overtube 300 from the distal end side in the non-mounting state of the inner needle 600.

As illustrated in these drawings., the distal end cap 360 of the overtube 300 has a columnar through-hole 364 that forms a portion of the treatment tool insertion passage 308 and through which the long needle part 602 is inserted in the mounting state between the overtube 300 and the inner needle 600, and a columnar through-hole 362 that forms a portion of the endoscope insertion passage 306 through which the short needle part 610 is inserted in the mounting state between the overtube 300 and the inner needle 600.

Additionally, the distal end cap 360 has the distal end surface 304 based on a tapered shape that protrudes from a point of an outer peripheral edge of a distal end of the long tubular overtube part 320 toward a right position (a point on the treatment tool insertion axis 308a) of the reference axis 300a on the distal end side of the point, that is, a tapered part that is tapered toward the distal end, and the second distal end opening 316 that is a distal end opening of the through-hole 364, and the first distal end opening 312 that is a distal end opening of the through-hole 362 are formed in the distal end surface 304.

The second distal end opening 316 is formed at a position closer to the right than the reference axis 300a because a center position thereof is a passage position of the treatment tool insertion axis 308a, and is formed at a position where the second distal end opening protrudes further to a front side (distal end side) than the first distal end opening 312.

Additionally, the second distal end opening 316 is open in a direction perpendicularly to the reference axis 300a. That is, a circular surface of an intersecting portion between the plane substantially perpendicular to the reference axis 300a and the treatment tool insertion axis 308a and the through-hole 364 is given as an opening surface. Hence, the opening surface of the second distal end opening 316 is substantially orthogonal to the reference axis 300a and the treatment tool insertion axis 308a.

The first distal end opening 312 is formed at a position closer to the left than the reference axis 300a because a center position thereof is a passage position of the endoscope insertion axis 306a, and is formed at a position where the first distal end opening protrudes further to a rear side (proximal end side) than the second distal end opening 316.

Additionally, the first distal end opening 312 is open in a direction oblique to the reference axis 300a. That is, an elliptical surface of an intersecting portion between a plane obliquely intersecting the reference axis 300a and the endoscope insertion axis 306a and the through-hole 362 along the inclination of the distal end surface 304 is give as an opening surface. For example, the opening surface follows a plane that is perpendicular to the horizontal reference plane along the leftward-rightward direction including the reference axis 300a and obliquely intersects the vertical reference plane along the upward-downward direction including the reference axis 300a from a front right side toward a rear left side. Hence, the opening surface of the first distal end opening 312 is inclined with a side near the reference axis 300a and the treatment tool insertion axis 308a as the front side.

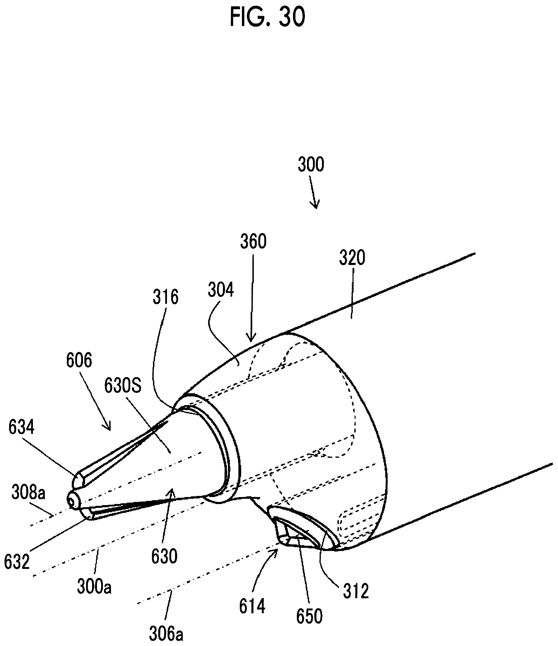

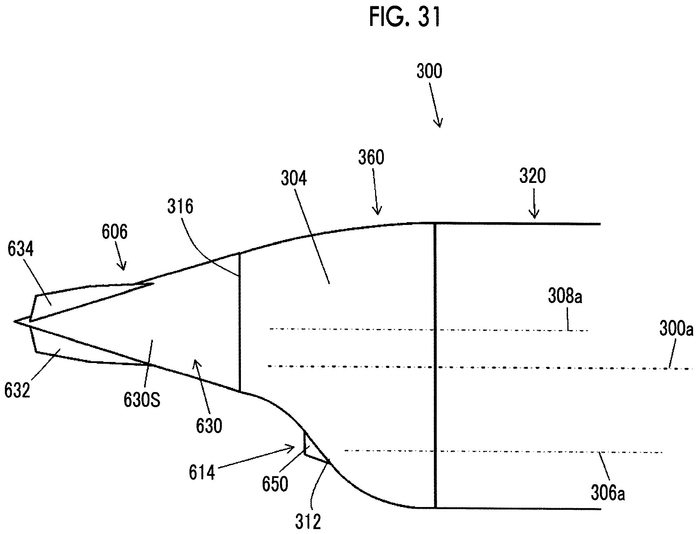

Figs. 30, 31, and 32 are respectively a perspective views, a plan view, and a front view illustrating the distal end portion of the overtube 300 in a state where the inner needle 600 is mounted on the overtube 300 of Figs. 27, 28, and 29.

As illustrated in these drawings, in the mounting state between the overtube 300 and the inner needle 600, the distal end part 606 of the long needle part 602 of the inner needle 600 is disposed to protrude from the second distal end opening 316 in the distal end cap 360 of the overtube 300 to the distal end side, and the distal end part 614 of the short needle part 610 of the inner needle 600 is disposed to protrude from the first distal end opening 312 to the distal end side.

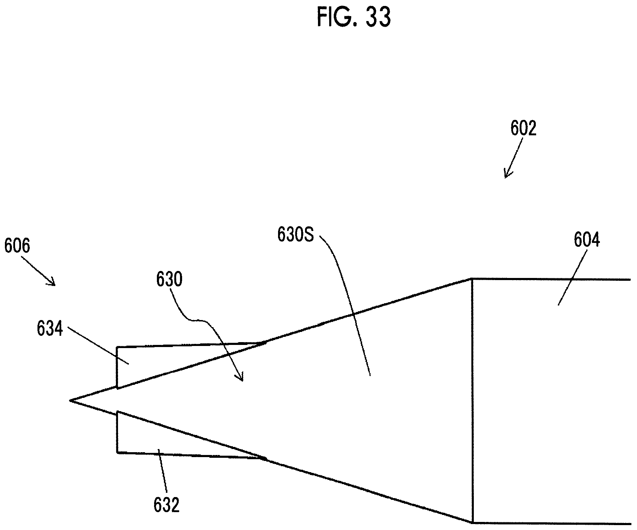

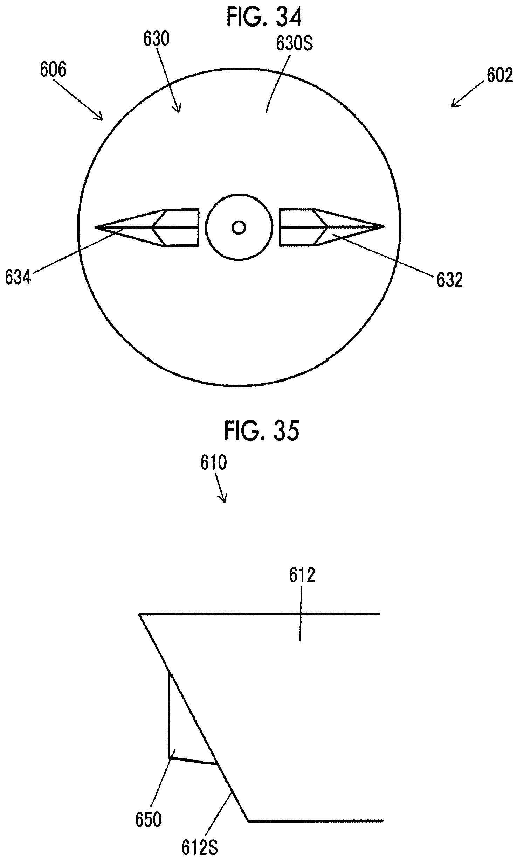

Here, Fig. 33 is a plan view illustrating the periphery of the distal end part 606 of the long needle part 602 from the upper side, and Fig. 34 is a front view illustrating the periphery of the distal end part 606 from the distal end side.

As illustrated in these drawings, the distal end part 606 of the long needle part 602 is continuously installed on the distal end side from the position of a circular outer peripheral edge of the distal end of the shaft part 604, and has a needle tip part 630 that has a tapered shape (conical shape) consisting of an inclined surface 630S tapered toward a distal end and of which the distal end is rounded off.

Additionally, a pair of cutting blade parts 632 and 634, which extends linearly from a position slightly closer to the proximal end side than the distal end to the rear side (proximal end side) along the inclined surface 630S, is provided as a second cutting blade part in the inclined surface 630S of the needle tip part 630.

The cutting blade parts 632 and 634 are disposed at positions that become symmetrical to each other with respect to a central axis of the long needle part 602 (shaft part 604).

Additionally, the cutting blade parts 632 and 634 are disposed to protrude in the shape of a thin plate at positions along a plane that is parallel to the reference axis 300a (600a) and passes through the position along the plane of the central axis of the long needle part 602 and the position of a central axis of the short needle part 610 in the distal end portion of the overtube 300. In addition, in the present embodiment, the central axis of the long needle part 602 and the central axis of the short needle part 610 are parallel to the reference axis 300a. Therefore, the cutting blade parts 632 and 634 are disposed to protrude in the shape of a thin plate at the positions along the plane including the central axis of the long needle part 602 and the central axis of the short needle part 610.

Accordingly, the cutting blade parts 632 and 634 have length components orthogonal to the central axis of the long needle part 602.

In the mounting state between the overtube 300 and the inner needle 600, as illustrated in Figs. 30 to 32, the distal end of the shaft part 604 is disposed at a position that substantially coincides with the opening surface of the second distal end opening 316, and the distal end part 606 consisting of the needle tip part 630 and the cutting blade parts 632 and 634 is disposed to protrude from the opening surface of the second distal end opening 316 to the distal end side. In this case, the cutting blade parts 632 and 634 have length components orthogonal to the reference axis 300a of the overtube 300.

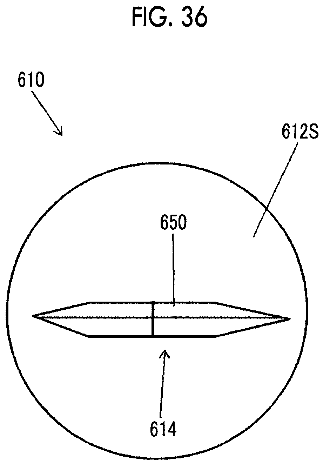

Here, Fig. 35 is a plan view illustrating the periphery of the distal end part 614 of the short needle part 610 from the upper side, and Fig. 36 is a front view illustrating the periphery of the distal end part 614 from the distal end side.

As illustrated in these drawings, a flat distal end surface 612S which is obliquely inclined with respect to the central axis of the shaft part 612 is provided at the distal end of the shaft part 612 of the short needle part 610.

The cutting blade part 650 that extends linearly along the distal end surface 612S is provided as a first cutting blade part on the distal end surface 612S.

Additionally, the cutting blade part 650 is disposed at a position along the same plane together with the cutting blade parts 632 and 634 of the long needle part 602 at the distal end portion of the overtube 300, and is disposed to protrude in the shape of a thin plate at a position along the plane that is parallel to the reference axis 300a (600a) and passes through the position of the central axis of the long needle part 602 and the position of the central axis of the short needle part 610. In the present embodiment, the central axis of the long needle part 602 and the central axis of the short needle part 610 are parallel to the reference axis 300a. Therefore, the cutting blade part 650 is disposed to protrude in the shape of a thin plate at the position along the plane including the central axis of the long needle part 602 and the central axis of the short needle part 610.

Accordingly, the cutting blade part 650 has a length component orthogonal to the central axis the short needle part 610.

Additionally, as illustrated in Fig. 35, the cutting blade part 650 protrudes in a triangular shape as seen from the upper side, and is formed such that a vertex protruding from the distal end surface 612S is present at substantially the same position in the forward-backward direction as a vertex on the distal end side out of the other two vertices on the distal end surface 612S.

Also, in the mounting state between the overtube 300 and the inner needle 600, the distal end surface 612S of the shaft part 612 is disposed along the opening surface of the first distal end opening 312, and the distal end part 614 (cutting blade part 650) of the short needle part 610 is disposed to protrude from the opening surface of the first distal end opening 312 to the distal end side. In this case, the cutting blade part 650 has a length component orthogonal to the reference axis 300a of the overtube 300, similar to the cutting blade parts 632 and 634 of the long needle part 602.

Additionally, since the cutting blade parts 632 and 634 of the long needle part 602 and the cutting blade part 650 of the short needle part 610 are formed at the positions along the same plane including the central axis of the long needle part 602 and the central axis of the short needle part 610, the cutting blade parts 632 and 634 and the cutting blade part 650 are disposed along the same straight line as each other, more specifically, on the same straight line as each other when the cutting blade parts 632 and 634 and the cutting blade part 650 are projected on the plane perpendicular to the reference axis 300a.

In addition, the distal end surface 612S of the shaft part 612 being disposed along the opening surface of the first distal end opening 312 includes not only a case where the distal end surface 612S and the opening surface of the first distal end opening 312 become parallel to each other and flush with each other, but also a case where the distal end surface 612S is disposed at a position closer to the proximal end side or the distal end side than the opening surface of the first distal end opening 312 or a case where the distal end surface and the opening surface are substantially parallel to each other.

According to the configuration of the distal end portion and the inner needle 600 of the above overtube 300, the distal end portion of the overtube 300 (and the inner needle 600) in a state where the inner needle 600 is mounted has a tapered shape (conical shape) as a whole by the distal end surface 304 (distal end cap 360) of the overtube 300, the needle tip part 630 (inclined surface 630S) of the distal end part 606 of the long needle part 602, and the distal end surface 612S of the shaft part 612 of the short needle part 610.

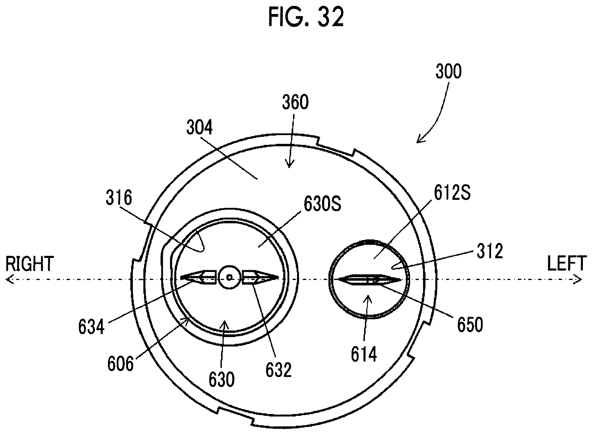

Additionally, in a case where the distal end portion of the overtube 300 is projected on the plane perpendicular to the reference axis 300a, that is, if the distal end portion of the overtube 300 is seen in a plan view from the distal end side, as illustrated in Fig. 32, the cutting blade parts 632 and 634 of the distal end part 606 of the long needle part 602 and the cutting blade part 650 of the distal end part 614 of the short needle part 610 are disposed at positions along the same straight line as each other.

Accordingly, the shape of the distal end portion of the overtube 300 (and the inner needle 600) in a state where the inner needle 600 is mounted has a shape similar to the distal end portion of the overtube in a state where the inner needle is mounted having one needle part in the overtube having one insertion passage.

For that reason, regarding the insertion power required in a case where a body wall is punctured with the overtube 300 or the penetrating power required in a case where the overtube 300 passes through a body wall, there is little influence of the number of insertion passages of the overtube 300 being two, the required insertion power and penetrating power can be made small, and the puncturing of the overtube 300 can be easily performed.

Additionally, since the cutting blade parts 632, 634, and 650 are linearly disposed, the task of tearing a body wall apart is not greatly impaired, and the insertion load of the body wall, the inner needle 600, and the overtube 300 when the body wall is punctured with the overtube 300 can be reduced.

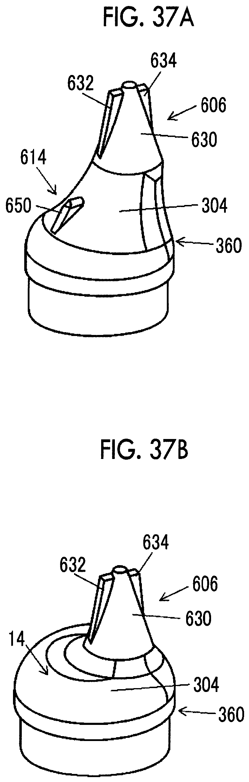

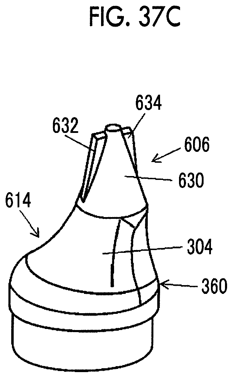

Three embodiments in which the shapes of distal end portions of the overtube 300 are different from each other in the mounting state between the overtube 300 and the inner needle 600 are illustrated in Fig. 37A to Fig. 37C. Fig. 37A illustrates the present embodiment. Fig. 37B illustrates a first comparison embodiment that is different from the present embodiment in that the distal end surface 304 of the overtube 300 is flat and in that the short needle part 610 of the inner needle 600 does not have the distal end part 614 (cutting blade part 650). Fig. 37C illustrates a second comparison embodiment that is different from the present embodiment in that the short needle part 610 of the inner needle 600 does not have the distal end part 614 (cutting blade part 650).

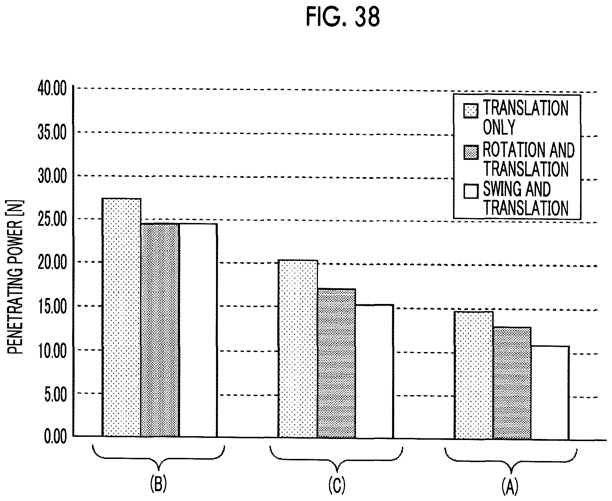

Meanwhile, the magnitudes of penetrating power required in a case where a body wall is punctured with these three embodiments, respectively, are illustrated as bar graphs in Fig. 38. A graph illustrated by portion (A) of Fig. 38 illustrates the penetrating power of the present embodiment, a graph illustrated by portion (B) of Fig. 38 illustrates the penetrating power of the first comparison embodiment, and a graph illustrated by portion (C) of Fig. 38 illustrates the penetrating power of the second comparison embodiment. Additionally, the case of a first condition under which puncturing is performed only by the forward movement (translation) of the overtube 300 in the axial direction (reference axis 300a), the case of a second condition under which puncturing is performed by the rotation of the overtube 300 around the axis together with the forward movement (translation) of the overtube 300 in the axial direction, and the case of a third condition under which puncturing is performed by the swing of the overtube 300 in the leftward-rightward direction orthogonal to the axis together with the forward movement (translation) of the overtube 300 in the axial direction are respectively illustrated sequentially from the left as the penetrating powers of the respective embodiments in portion (A) to portion (C) of Fig. 38.

As illustrated by portion (A) to portion (C) of Fig. 38, even in any embodiments, the puncturing under the first condition requires the largest penetrating power, and in the present embodiment and the second comparison embodiment, the puncturing under the third condition requires the smallest penetrating power. In the first comparison embodiment, the puncturing under the second condition and the third condition requires substantially the same degree of penetrating power.

Meanwhile, in a case where the conditions of puncturing are fixed, even in any of the first condition to the third condition, the penetrating powers become smaller in order of the first comparison embodiment, the second comparison embodiment, and the present embodiment. Additionally, in the present embodiment, the penetrating powers under all the conditions are smaller than the penetrating powers under any conditions in the first comparison embodiment and the second comparison embodiment.

As can be seen from such comparison, the penetrating powers are reduced as in the second comparison embodiment and the present embodiment with respect to the first comparison embodiment by using the tapered shape (conical shape) as the shape of the distal end portion of the overtube 300 in the mounting state between the overtube 300.

Additionally, by forming the cutting blade parts 632, 634, and 650 in the distal end part 606 of the long needle part 602 and the distal end part 614 of the short needle part 610 along the same straight line, the penetrating powers are reduced as in the present embodiment with respect to the second comparison embodiment.

In addition, since a difference is caused in penetrating power even with a difference of whether or not the short needle part 610 has the cutting blade part 650 as in a case where the cutting blade part 650 of the short needle part 610 is provided with respect to the first comparison embodiment of Fig. 37B and the short needle part 610 has the cutting blade part 650, there is an effect of reducing the penetrating power.

Subsequently, other forms of the distal end part 614 of the short needle part 610 of the inner needle 600 will be described.

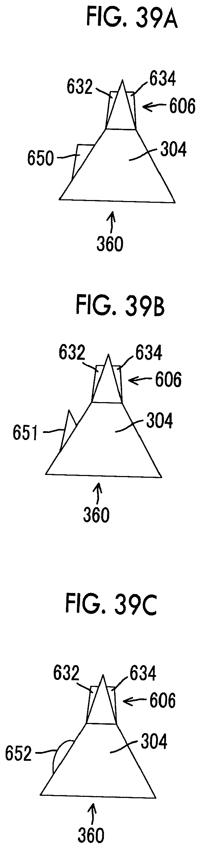

Fig. 39A to Fig. 39E are plan views or side views illustrating the shapes of the distal end portion of the overtube 300 in the mounting state between the overtube 300 and the inner needle 600 in a simplified manner from an upper side or a left side, and illustrated a plurality of different forms of first cutting blade parts formed in the distal end part 614 of the short needle part 610.

Fig. 39A illustrates a case where a first cutting blade part formed in the distal end part 614 of the short needle part 610 is equivalent to the cutting blade part 650 of the above embodiment. When the first cutting blade part, and the second cutting blade part (cutting blade parts 632 and 634) formed in the distal end part 606 of the long needle part 602 are projected on the plane perpendicular to the reference axis 300a, the cutting blade part 650 that is the first cutting blade part and the cutting blade parts 632 and 634 that are the second cutting blade part are disposed along the same straight line. Additionally, the cutting blade part 650 protrudes in a triangular shape with respect to the distal end surface 304 of the overtube 300, and a vertex that protrudes from the distal end surface 304 substantially coincides with the position of a vertex on the distal end side out of the other two vertices in terms of positions in the forward-backward direction.

In contrast, as long as the first cutting blade part formed in the distal end part 614 of the short needle part 610 is disposed along the same straight line as the second cutting blade part (cutting blade parts 632 and 634) formed in the distal end part 606 of the long needle part 602 when being projected on the plane perpendicular to the reference axis 300a, the forms as illustrated in Fig. 39B to Fig. 39E may be adopted. Even in the forms as illustrated in Fig. 39B to Fig. 39E, an advantageous effect that it is possible to reduce the insertion load without greatly impairing a tearing action onto a body wall can be obtained.

In addition, the first cutting blade part and the second cutting blade part being disposed along the same straight line includes not only a case where the first cutting blade part and the second cutting blade part are on the same straight line, but also a case where the first cutting blade part and the second cutting blade part are not on the same straight line but are parallel to each other and a case where the first cutting blade part and the second cutting blade part are not on the same straight line and are not parallel to each other but are substantially parallel to each other.

The form of Fig. 39B is different from the configuration of Fig. 39A in terms pf a protruding shape of a first cutting blade part 651 formed in the distal end part 614 of the short needle part 610. Although the first cutting blade part 651 protrudes in a triangular shape with respect to the distal end surface 612S (the opening surface of the first distal end opening 312) of the shaft part 612 as in Fig. 39A, a vertex of the first cutting blade part 651 that protrudes from the distal end surface 612S protrudes further to the distal end side than the other two vertices on the distal end surface 612S.

The form of Fig. 39C is different from the form of Fig. 39A in terms pf a protruding shape of a first cutting blade part 652 formed in the distal end part 614 of the short needle part 610. The first cutting blade part 652 protrudes in a circular-arc shape with respect to the distal end surface 612S (the opening surface of the first distal end opening 312).

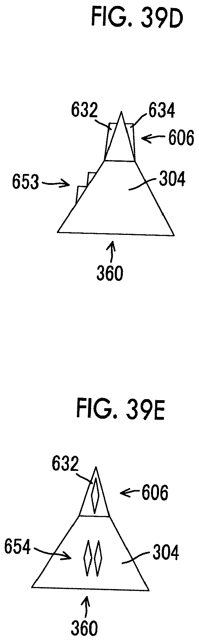

The form of Fig. 39D is different from the form of Fig. 39A in terms pf the number of blades of a first cutting blade part 653 formed in the distal end part 614 of the short needle part 610. Two cutting blades having the same protruding shape as that of Fig. 39A are disposed in series on the first cutting blade part 653. However, the protruding shape of each cutting blade may be the same as that of Figs. 39B or 39C, or three or more cutting blades may be disposed in series.

Fig. 39E is a side view illustrating the distal end portion of the overtube 300 from the left side, and the form of Fig. 39E is different from the form of Fig. 39A in terms pf the number of blades of a first cutting blade part 654 formed in the distal end part 614 of the short needle part 610. Two cutting blades having the same protruding shape as that of Fig. 39A are disposed in parallel on the first cutting blade part 654. However, the protruding shape of each cutting blade may be the same as that of Figs. 39B or 39C, or three or more cutting blades may be disposed in parallel.

As described above, although the inner needle 600 of the above embodiment is used for the overtube 300 (hereinafter referred to as the overtube 300 of Fig. 4) of Figs. 1 to 19 where the endoscope insertion passage 306 and the treatment tool insertion passage 308 are disposed parallel to each other, an inner needle of substantially the same configuration can be used for the overtube 300 (hereinafter referred to as the overtube 300 of Fig. 20) illustrated in Figs. 20 to 23 where the endoscope insertion passage 306 and the treatment tool insertion passage 308 are disposed to obliquely intersect each other.

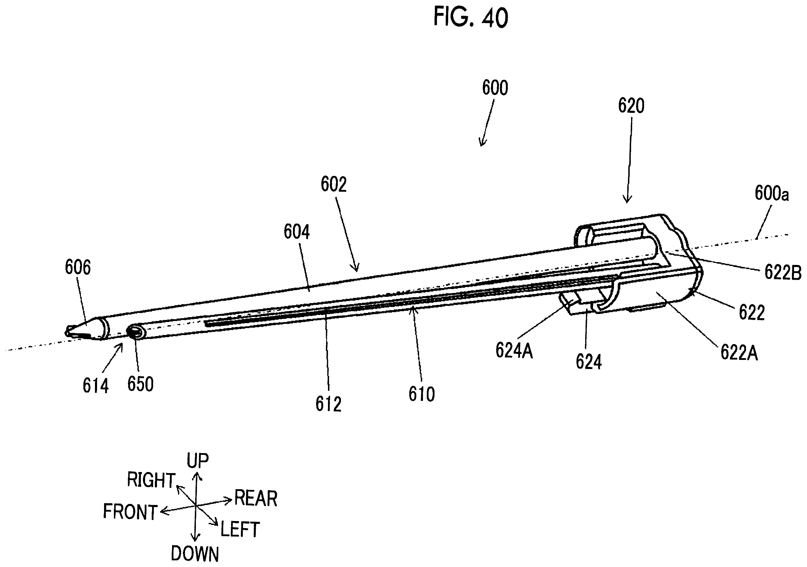

Fig. 40 illustrates the inner needle 600 used for the overtube 300 of Fig. 20. In the inner needle 600 of Fig. 40, the constituent elements of functions that are the same as or similar to those of the above inner needle 600 illustrated in Fig. 24 are designated by the same reference signs, but constituent elements that are different from those of Fig. 24 as members is not present.

Meanwhile, in the inner needle 600 of Fig. 40, in correspondence with the endoscope insertion passage 306 and the treatment tool insertion passage 308 of the overtube 300 of Fig. 20 obliquely intersecting each other, the long needle part 602 is parallel to the reference axis 600a, while the short needle part 610 obliquely intersects the reference axis 600a. Also, the long needle part 602 and the short needle part 610 is disposed in the mounting state between the overtube 300 of Fig. 20 and the inner needle 600 of Fig. 40 such that the central axis of the long needle part 602 is substantially coaxial with the treatment tool insertion axis 308a and such that the central axis of the short needle part 610 is substantially coaxial with the endoscope insertion axis 306a.

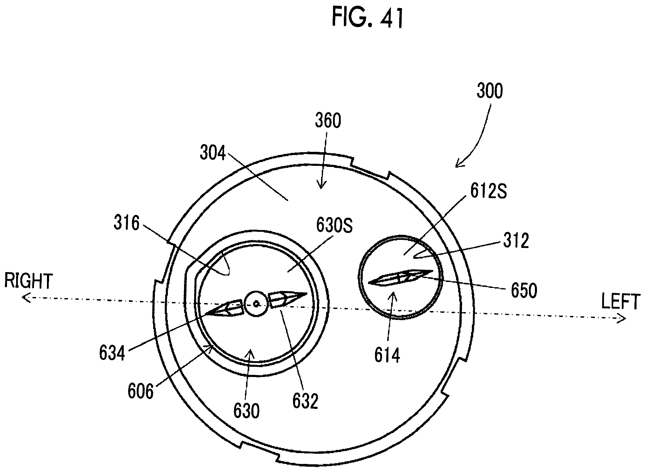

Additionally, Fig. 41 is a front view illustrating the distal end portion of the overtube 300 in the mounting state between the overtube 300 of Fig. 20 and the inner needle 600 of Fig. 40 from the distal end side. As illustrated in this Fig. (refer to Fig. 32), in the overtube 300 of Fig. 20, the position of the first distal end opening 312 is displaced to an upper side as compared to the overtube 300 of Fig. 4. Thus, the position of the distal end surface 612S of the shaft part 612 of the short needle part 610 is also present on the upper side with respect to the distal end part 606 of the long needle part 602.

In contrast, similar to the inner needle 600 of Fig. 24, the cutting blade parts 632 and 634 in the distal end part 606 of the long needle part 602 and the cutting blade part 650 in the distal end part 614 of the short needle part 610 are disposed to protrude in the shape of a thin plate at positions along the plane that is parallel to the reference axis 300a (600a) and passes through the position along the plane of the central axis of the long needle part 602 and the position of the central axis of the short needle part 610 in the distal end portion of the overtube 300.

Accordingly, when the cutting blade parts 632 and 634 and the cutting blade part 650 are projected on the plane perpendicular to the reference axis 300a, the cutting blade parts 632 and 634 and the cutting blade part 650 are disposed along the same straight line.

### Explanation of References

- 10:: endoscopic surgical device
- 100:: endoscope
- 102:: endoscope insertion part
- 200:: treatment tool
- 202:: treatment tool insertion part
- 300:: overtube
- 300a, 600a:: reference axis
- 302:: proximal end surface
- 306:: endoscope insertion passage
- 306a:: endoscope insertion axis
- 308:: treatment tool insertion passage
- 308a:: treatment tool insertion axis
- 310:: first proximal end opening
- 312:: first distal end opening
- 314:: second proximal end opening
- 316:: second distal end opening
- 320:: long tubular overtube part
- 324:: partition wall member
- 326:: endoscope guide groove
- 328:: treatment tool guide groove
- 340:: proximal end cap
- 360:: distal end cap
- 400:: slider
- 402:: coupling ring
- 430:: endoscope fixing tool
- 450:: treatment tool fixing tool
- 500:: sheathing tube
- 600:: inner needle
- 602:: long needle part
- 604, 612:: shaft part
- 606, 614:: distal end part
- 610:: short needle part
- 620:: head part
- 624:: locking lever
- 630:: needle tip part
- 630S:: inclined surfaces
- 632, 634, 650, 651, 652, 653, 654:: cutting blade part

## Claims

1. An endoscopic surgical device comprising:
an overtube; and
an inner needle inserted through the overtube,
the device puncturing a body wall in a state where the overtube and the inner needle are combined together,
wherein the overtube includes
an overtube body having a distal end, a proximal end, and a longitudinal axis,
a first distal end opening and a second distal end opening provided at a distal end of the overtube body,
a first proximal end opening and a second proximal end opening provided at a proximal end of the overtube body,
a first insertion passage that is provided along the longitudinal axis of the overtube body and allows the first distal end opening and the first proximal end opening to communicate with each other,
a second insertion passage that is provided along the longitudinal axis of the overtube body and allows the second distal end opening and the second proximal end opening to communicate with each other, and
wherein the inner needle includes
a first needle part that has a first distal end part and is inserted through the first insertion passage,
a second needle part that has a second distal end part and is inserted through the second insertion passage,
a first cutting blade part that is formed at the first distal end part, and has a length component orthogonal to the longitudinal axis,
a second cutting blade part that is formed at the second distal end part and has a length component orthogonal to the longitudinal axis, and
a positioning part that defines a position of the first distal end part with respect to the first distal end opening and a position of the second distal end part with respect to the second distal end opening in a state where the overtube and the inner needle are combined together, and
wherein the first cutting blade part and the second cutting blade part are disposed along the same straight line as each other when the first cutting blade part and the second cutting blade part are projected on a plane perpendicular to the longitudinal axis.

2. The endoscopic surgical device according to claim 1,
wherein the first cutting blade part and the second cutting blade part are disposed on the same straight line as each other when the first cutting blade part and the second cutting blade part are projected on the plane perpendicular to the longitudinal axis.

3. The endoscopic surgical device according to claim 1 or 2,
wherein the overtube body has a tapered part that is tapered toward a distal end, and
wherein the tapered part has the second distal end opening, and the first distal end opening disposed closer to a proximal end side than the second distal end opening.

4. The endoscopic surgical device according to claim 3,
wherein the second distal end opening is open in a direction perpendicular to the longitudinal axis, and
wherein the first distal end opening is open in a direction oblique to the longitudinal axis.

5. The endoscopic surgical device according to any one of claims 1 to 4,
wherein the second distal end part has an inclined surface that is tapered toward the distal end, and the inclined surface is provided at a position where the inclined surface protrudes from the second distal end opening when being positioned by the positioning part, and
wherein a pair of the second cutting blade parts is provided on the inclined surface, and the pair of second cutting blade parts are disposed at positions that become symmetrical to each other with respect to a central axis of the second needle part.

6. The endoscopic surgical device according to any one of claims 1 to 5,
wherein the first distal end part has a distal end surface disposed along an opening surface of the first distal end opening when being positioned by the positioning part, and
wherein the first cutting blade part is provided on the distal end surface.

7. The endoscopic surgical device according to any one of claims 1 to 6,
wherein the overtube includes an interlocking member that is movable forward and backward inside the overtube body, and
wherein the interlocking member has a first coupling part coupled to a first insertion part of a first medical instrument inserted through the first insertion passage, and a second coupling part coupled to a second insertion part of a second medical instrument inserted through a second insertion passage.

8. The endoscopic surgical device according to claim 7,
wherein the interlocking member has a non-sensing region where the forward and backward movement of any one of the first insertion part and the second insertion part does not interlock with the forward and backward movement of the other of the first insertion part and the second insertion part, and a sensing region where the forward and backward movement of any one of the first insertion part and the second insertion part interlocks with the forward and backward movement of the other of the first insertion part and the second insertion part.

9. The endoscopic surgical device according to any one of claims 1 to 8,
wherein the first insertion passage is an endoscope insertion passage through which an endoscope is inserted, and
wherein the second insertion passage is a treatment tool insertion passage through which a treatment tool is inserted.

10. The endoscopic surgical device according to claim 9,
wherein an internal diameter of the second insertion passage is larger than an internal diameter of the first insertion passage.
